(19) Europäisches Patentamt
European Patent Office
Office européen des brevets

(11) Veröffentlichungsnummer : **0 410 278 B1**

(12) # EUROPÄISCHE PATENTSCHRIFT

(45) Veröffentlichungstag der Patentschrift : **19.01.94 Patentblatt 94/03**

(21) Anmeldenummer : **90113712.5**

(22) Anmeldetag : **18.07.90**

(51) Int. Cl.$^5$ : **C07K 5/06,** C07D 233/64, C07D 401/12, C07D 409/12, C07D 403/12, A61K 31/415, A61K 31/445, A61K 31/535, A61K 37/64, // (C07D401/12, 233:64, 211:58), (C07D409/12, 233:64, 333:24), (C07D403/12, 233:64, 209:18)

(54) **Renin-hemmende Aminooligohydroxy-Derivate.**

(30) Priorität : **25.07.89 DE 3924506**
**30.09.89 DE 3932817**

(43) Veröffentlichungstag der Anmeldung : **30.01.91 Patentblatt 91/05**

(45) Bekanntmachung des Hinweises auf die Patenterteilung : **19.01.94 Patentblatt 94/03**

(84) Benannte Vertragsstaaten : **AT BE CH DE DK ES FR GB GR IT LI LU NL SE**

(56) Entgegenhaltungen :
**EP-A- 0 189 203**
**EP-A- 0 230 266**
**US-A- 4 863 905**

(73) Patentinhaber : **HOECHST AKTIENGESELLSCHAFT**
**D-65926 Frankfurt (DE)**

(72) Erfinder : **Kleemann, Heinz-Werner, Dr.**
**Jahnstrasse 6**
**D-6092 Kelsterbach (DE)**
Erfinder : **Urbach, Hansjörg, Dr.**
**Le Lavandoustrasse 41**
**D-6242 Kronberg/Taunus (DE)**
Erfinder : **Wagner, Adalbert, Dr.**
**Kleiststrasse 9**
**D-6238 Hofheim am Taunus (DE)**
Erfinder : **Ruppert, Dieter, Dr.**
**Schreyerstrasse 30**
**D-6242 Kronberg/Taunus (DE)**
Erfinder : **Linz, Wolfgang, Dr.**
**Huxelrebenweg 54**
**D-6500 Mainz (DE)**
Erfinder : **Kramer, Werner, Dr. Dr.**
**Henry Moisand 19**
**D-6500 Mainz (DE)**

**EP 0 410 278 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aus den europäischen Patentanmeldungen EP-A 184 855, 189 203, 202 577, 229 667, 230 266 sowie 237 202 und der International Patent Application WO 87/05302 sind Aminodiol-Derivate mit reninhemmender Wirkung bekannt.

Weiterhin sind reninhemmende Aminodiol-Derivate in Biochem. Biophys. Res. Commun. 132, 155 - 161 (1985), in Biochem. Biophys. Res. Commun. 146, 959 - 963 (1987), in FEBS Lett. 230, 38 - 42 (1988) und in J. Med Chem. 30, 976 - 982 (1987) beschrieben.

Überraschenderweise wurde nun gefunden, daß solche Verbindungen, die sich von den in den genannten Dokumenten beschriebenen dadurch unterscheiden, daß sie am C-Terminus zusätzliche Hydroxyfunktionen enthalten, hochwirksame Reninhemmer in vitro und in vivo sind und gegenüber den bekannten Verbindungen vorteilhafte Eigenschaften aufweisen.

Die Erfindung betrifft Verbindungen der Formel I

$$A - \overset{R^2}{\overset{|}{N}} - \overset{R^3}{\overset{|}{CH}} - CO - NH - \overset{R^4}{\overset{|}{CH}} - (CHOH)_n - CH_2OH \qquad (I)$$

in welcher

A einen Rest der Formeln II, III oder IV bedeutet

$$R^1 - \overset{R^6}{\overset{|}{N}} - \overset{R^5}{\overset{|}{CH}} - \overset{O}{\overset{\|}{C}} - \qquad (II)$$

$$R^1 - \overset{R^7}{\overset{|}{CH}} - \overset{R^5}{CH} - \overset{O}{\overset{\|}{C}} - \qquad (III)$$

$$R^1 - O - \overset{R^5}{\overset{|}{CH}} - \overset{O}{\overset{\|}{C}} - \qquad (IV)$$

$R^1$ Wasserstoff, ($C_1$-$C_8$)-Alkylsulfonyl; ($C_1$-$C_8$)-Alkylsulfinyl; 2-Hydroxyethylsulfonyl; 2-Hydroxypropylsulfonyl; 2-Hydroxypropionyl; 3-Hydroxypropionyl; 3-Hydroxybutyryl; 2-Hydroxy-3-methylbutyryl; ($C_1$-$C_8$)-Alkanoyloxy-($C_1$-$C_{10}$)-alkyl; n-Decanoyl; Formyl; Acetyl; Propionyl; Pivaloyl; Isovaleryl; Isobutyryl; (3-Amino-3,3-dimethyl)-propionyl; 4-Aminobutyryl; 5-Aminopentanoyl; 6-Aminohexanoyl; Dimethylaminoacetyl; Piperidino-4-carbonyl; Morpholino-4-carbonyl; Cyclopropylcarbonyl; Cyclobutylcarbonyl; Cyclopentylcarbonyl; Cyclohexylcarbonyl; 3-Aminocyclobutylcarbonyl; 4-Aminocyclohexylcarbonyl; 3-Aminocyclobutylsulfonyl; 4-Aminocyclohexylsulfonyl; Phenylacetyl; Phenylpropanoyl; Phenylbutanoyl; 2-Pyridyl-($C_1$-$C_8$)-alkanoyl; 3-Pyridyl-($C_1$-$C_8$)-alkanoyl; 4-Pyridyl-($C_1$-$C_8$)-alkanoyl; 4-Chlorbenzoyl; 4-Methylbenzoyl; 2-Methoxycarbonylbenzoyl; 4-Methoxybenzoyl; Pyrrolyl-2-carbonyl; Pyridyl-3-carbonyl; Benzolsulfonyl; Methoxycarbonyl; Ethoxycarbonyl; Isobutoxycarbonyl; tert.-Butoxycarbonyl; 2-(Trimethylsilyl)-ethoxycarbonyl; 2,2,2-Trichlorethoxycarbonyl; 1,1-Dimethyl-2,2,2-trichlorethoxycarbonyl; Benzyloxy-carbonyl; 1- oder 2-Naphthylmethoxycarbonyl; 9-Fluorenylmethoxycarbonyl; 4-Amino-piperidino-1-carbonyl; 4-Aminomethyl-piperidino-1-carbonyl; N-Methyl-[2-<N-(morpholinocarbonyl)-N-methylamino>-ethyl]-aminocarbonyl, oder N-(4-piperidino)-carbamoyl bedeutet;

$R^1$ und $R^5$ zusammen mit dem sie tragenden Stickstoffatom einen 8- bis 12-gliedrigen bicyclischen Ring bilden, der aromatisch, teilhydriert oder vollständig hydriert sein kann;

$R^2$ und $R^6$ unabhängig voneinander Wasserstoff oder Methyl bedeuten;

$R^3$ und $R^5$ unabhängig voneinander Wasserstoff, Methyl, Ethyl, Isopropyl, n-Propyl, n-Butyl, Isobutyl, sec.-

Butyl, 3-Guanidinopropyl, Carbamoylmethyl, 2-Carbamoylethyl, Carboxymethyl, 2-Carboxyethyl, Mercaptomethyl, 2-(Methylthio)-ethyl, (1-Mercapto-1-methyl)-ethyl, Hydroxymethyl, 1-Hydroxyethyl, Aminomethyl, 2-Aminoethyl, 3-Aminopropyl, 4-Aminobutyl, N,N-Dimethylamino, Cyclohexylmethyl, Imidazol-4-yl-methyl, Benzyl, 2-Methyl-benzyl, 3-Methylbenzyl, Indol-3-yl-methyl, 4-Hydroxybenzyl, 4-Methoxybenzyl, 3,4-Dihydroxybenzyl, 3,4-Dimethoxybenzyl, (Benzdioxolan-5-yl)-methyl, 2-Thienyl, 2-Thienylmethyl, 2-(2-Thienyl)-ethyl, 3-Thienyl, 3-Thienylmethyl, 2-(3-Thienyl)-ethyl, 4-Chlorbenzyl, 2-(Methylsulfinyl)-ethyl, 2-(Methylsulfonyl)-ethyl, 2-Pyridylmethyl, 3-Pyridylmethyl, 4-Pyridylmethyl, Cyclohexyl, (1-Methyl-imidazol-4-yl)-methyl, (3-Methyl-imidazol-4-yl)-methyl, Phenyl, 1-Naphthylmethyl, 2-Naphthylmethyl, 2-Phenylethyl, 2-Thiazolylmethyl, 4-Thiazolylmethyl, 3-Pyrazolylmethyl, 4-Pyrimidinylmethyl, Indol-2-yl-methyl, 2-Benzo[b]thienylmethyl, 3-Benzo[b]thienylmethyl, 2-Furylmethyl, Cyclohexyl oder Cyclopentyl bedeuten;

$R^4$ ($C_3$-$C_{12}$)-Alkyl; mono- oder bicyclisches ($C_3$-$C_{12}$)-Cycloalkyl oder ($C_3$-$C_{12}$)-Cycloalkylmethyl, wobei der Cycloalkylteil gegebenenfalls durch ($C_1$-$C_4$)-Alkyl substituiert ist; ($C_6$-$C_{10}$)-Aryl-methyl; Dithiolanyl; Dithiolanylmethyl; Dithianyl oder Dithianylmethyl bedeutet;

$R^7$ Wasserstoff bedeutet oder zusammen mit $R^1$ und den diese tragenden Atomen ein mono- oder bicyclisches gesättigtes oder teilweise ungesättigtes Ringsystem mit 5 - 12 Ringgliedern, das außer Kohlenstoff noch 1 Schwefelatom enthält, welches besonders bevorzugt zum Sulfon oxidiert ist bildet, oder zusammen mit $R^5$ und den diese tragenden Atome ein Thiochromansystem, dessen Schwefelatom besonders bevorzugt zum Sulfon oxidiert ist, bildet und

n 3 - 6 bedeutet, sowie deren physiologisch verträglichen Salze.

Die Chiralitätszentren in den Verbindungen der Formel I können die R-, S- oder R-S-Konfiguration aufweisen.

Alkyl kann geradkettig oder verzweigt sein. Entsprechendes gilt für davon abgeleitete Reste.

Unter ($C_3$-$C_8$)-Cycloalkyl werden Cyclopropyl, Cyclobutyl, Cyclopentyl, Cyclohexyl, Cycloheptyl und Cyclooctyl verstanden.

Unter ($C_4$-$C_{10}$)-Bicycloalkyl bzw. ($C_8$-$C_{12}$)-Tricycloalkyl versteht man einen isocyclischen aliphatischen, nicht aromatischen Rest, der gegebenenfalls unsymmetrisch verteilte Doppelbindungen enthalten kann, gegebenenfalls auch mit offenkettigen aliphatischen Seitenketten substituiert sein kann. Die zwei oder drei Ringe als Komponenten eines derartigen Restes sind kondensiert oder spiroverknüpft und über ein Ring-C-Atom oder ein Seitenketten-C-Atom verknüpft. Beispiele für diese Reste sind Bornyl-, Norbornyl-, Pinanyl-, Norpinanyl-, Caranyl-, Norcaranyl-, -Thujanyl-, Adamantyl-, Bicyclo(3.3.0)octyl-, Bicyclo(1.1.0)butyl-, Spiro(3.3)heptyl-Substituenten.

Falls die genannten Cyclen mehr als einen Substituenten tragen, so können diese sowohl cis als trans zueinander stehen.

($C_6$-$C_{14}$)-Aryl ist beispielsweise Phenyl, Naphthyl, Biphenylyl oder Fluorenyl; bevorzugt ist Phenyl. Entsprechendes gilt für davon abgeleitete Reste, wie z.B. Aryloxy, Aroyl, Aralkyl und Aralkyloxy. Unter Aralkyl versteht man einen mit Alkyl verknüpften unsubstituierten oder substituierten Aryl-Rest, wie z.B. Benzyl, α- und β-Naphthylmethyl, Halobenzyl und Alkoxybenzyl, wobei Aralkyl jedoch nicht auf die genannten Reste beschränkt wäre.

Ein Rest Het im Sinne vorstehender Definition ist beispielsweise Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzannelliertes, cyclopenta-, cyclohexa- oder cyclohepta-annelliertes Derivat dieser Reste. Dieser Heterocyclus kann an einem Stickstoffatom durch Oxido, ($C_1$-$C_6$)-Alkyl, z.B. Methyl oder Ethyl, Phenyl oder Phenyl-($C_1$-$C_4$)-alkyl, z.B. Benzyl, und/oder an einem oder mehreren Kohlenstoffatomen durch ($C_1$-$C_4$)-Alkyl, z.B. Methyl, Phenyl, Phenyl-($C_1$-$C_4$)-alkyl, z.B. Benzyl, Halogen, z.B. Chlor, Hydroxy, ($C_1$-$C_4$)-Alkoxy, z.B. Methoxy, Phenyl-($C_1$-$C_4$)-alkoxy, z.B. Benzyloxy, oder Oxo substituiert und teilweise gesättigt sein und ist beispielsweise 2- oder 3-Pyrrolyl, Phenyl-pyrrolyl, z.B. 4- oder 5-Phenyl-2-pyrrolyl, 2-Furyl, 2-Thienyl, 4-Imidazolyl, Methyl-imidazolyl, z.B. 1-Methyl-2-, 4- oder 5-imidazolyl, 1,3-Thiazol-2-yl, 2-, 3- oder 4-Pyridyl, 1-Oxido-2-, 3- oder 4-pyridyl, 2-Pyrazinyl, 2-, 4- oder 5-Pyrimidinyl, 2-, 3- oder 5-Indolyl, substituiertes 2-Indolyl, z.B. 1-Methyl-, 5-Methyl-, 5-Methoxy-, 5-Benzyloxy-, 5-Chlor- oder 4,5-Dimethyl-2-indolyl, 1-Benzyl-2- oder 3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl oder β-Carbolin-3-yl.

Teilhydrierte oder vollständig hydrierte heterocyclische Ringe sind beispielsweise Dihydropyridinyl, Pyrrolidinyl, z.B. 2-, 3- oder 4-N-Methylpyrrolidinyl, Piperidinyl, Piperazinyl, Morpholino, Thiomorpholino.

Halogen steht für Fluor, Chlor, Brom oder Jod, bevorzugt sind Fluor, Chlor und Brom.

Unter Salzen von Verbindungen der Formel I sind insbesondere pharmazeutisch verwendbare oder nicht-toxische Salze zu verstehen.

Solche Salzen werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel I mit einer endständigen Carboxylgruppe besitzen die nachstehenden Formeln VI und VII

A - OH (VI)

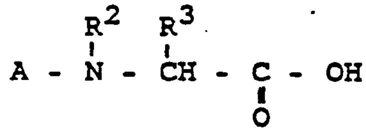

(VII)

Fragmente einer Verbindung der Formel I mit einer endständigen Aminogruppe besitzen die nachstehenden Formeln VIII bis X

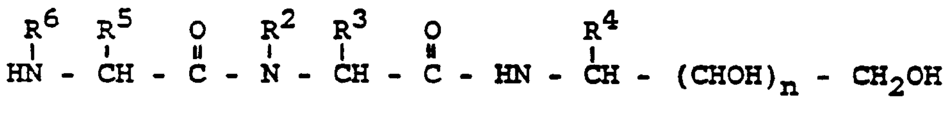

(VIII)

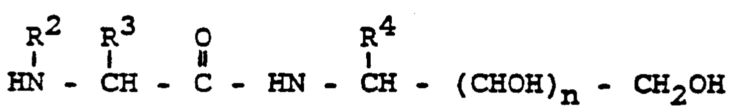

(IX)

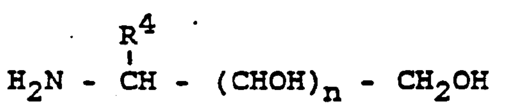

(X)

Methoden, die zur Herstellung einer Amidbindung geeignet sind, werden z.B. in Houben-Weyl, Methoden der organischen Chemie, Band 15/2; Bodanszky et al., Peptide Synthesis, 2nd ed. (Wiley & Sons, New York 1976) oder Gross, Meienhofer, The Peptides: Analysis, synthesis, biology (Academic Press, New York 1979) beschrieben. Vorzugsweise werden die folgenden Methoden herangezogen:

Aktivestermethode mit N-Hydroxy-succinimid, 1-Hydroxybenzotriazol oder 3-Hydroxy-4-oxo-3,4-dihydro-1,2,3-benzotriazin als Alkoholkomponente, Kupplung mit einem Carbodiimid wie Dicyclohexylcarbodiimid, mit Propanphosphonsäureanhydrid oder Methylethylphosphinsäureanhydrid und die Gemischt-Anhydrid-Methode mit Pivaloylchlorid oder Chlorameisensäureethylester oder -isobutylester, oder Kupplung mit Phosphonium-Reagenzien, wie Benzotriazol-1-yl-oxy-tris-(dimethylamino)-phosphonium-hexafluorophosphat (BOP) oder Uronium-Reagenzien, wie 2-(1H-Benzotriazol-1-yl)-1,1,3,3-tetramethyluronium-tetrafluoroborat (TBTU) oder 2-(1H-Benzotriazol-1-yl)-1,1,3,3-tetramethyluronium-hexafluorphosphat (z.B. Chem. Ber. 103 (1970) 788 u. 2034, Z. Naturforsch. 21b (1966) 426; Angew. Chem. Int. Ed. 19 (1980) 133; US-Patent 4,426,325)

Fragmente der Formel VI, sofern sie unter

a) Formel II fallen, werden nach den allgemein bekannten Methoden zur Herstellung von Aminosäuren synthetisiert;

b) Formel III fallen, werden entweder ausgehend von den entsprechenden α-Aminosäuren synthetisiert, wobei deren Chiralitätszentrum erhalten bleibt. Diazotierung bei -20°C bis 50°C in verd. Mineralsäuren

führt zu α-Bromcarbonsäuren oder über die Milchsäure zu α-Trifluormethansulfonyloxy-Carbonsäuren, die mit einem $R^1$ und $R^7$ tragenden Nucleophil umgesetzt werden können; oder

c) Formel IV fallen, werden ausgehend von den entsprechenden α-Aminosäuren synthetisiert, wobei deren Chiralitätszentrum erhalten bleibt. Diazotierung bei -20°C bis 50°C in verdünnten Mineralsäuren führt zu Milchsäuren, die mit einem $R^1$ tragenden Elektrophil umgesetzt werden können.

Fragmente der Formel VII werden nach den allgemein bekannten Methoden zur Herstellung von Aminosäuren und Peptiden synthetisiert.

Fragmente der Formel X werden aus geeigneten Kohlenhydraten über die entsprechenden geschützten γ-Lactone hergestellt. Zunächst erfolgt Umsetzung mit einem C-Nucleophil wie einer Grignard-Verbindung oder einer Alkyllithium-Verbindung, anschließend Aminoglykosidierung und reduktive Ringöffnung mit komplexen Hydriden wie $LiAlH_4$ oder katalytische Hydrierung.

Alternativ können-Fragmente der Formel X aus den geeigneten geschützten Aralkylaminoglycosiden hergestellt werden. Hierbei wird der Aralkylrest zweckmäßigerweise so gewählt, daß vor der Amidkopplung eine hydrogenolytische Entfernung möglich ist.

Umsetzung mit einem C-Nucleophil wie einer Alkyllithium-Verbindung in einem gegen diese Nucleophile inerten Lösungsmittel wie Diethylether, Tetrahydrofuran, Tetrahydropyran, Formaldehyddimethylacetal oder 1,2-Dimethoxyethan bei einer Temperatur zwischen -30°C und dem Siedepunkt des Lösungsmittels, bevorzugt zwischen 0°C und dem Siedepunkt des Lösungsmittels liefert das Aralkyl-Derivat des geschützten Fragments der Formel X. Dies kann durch katalytische Hydrierung mit Wasserstoff oder katalytische Transferhydrierung mit Ammoniumformiat zur Amidkopplung freigesetzt werden.

Die zur Herstellung von Verbindungen der Formel I erforderlichen Vor- und Nachoperationen wie Einführung und Abspaltung von Schutzgruppen sind literaturbekannt und sind z.B. in T.W. Greene "Protective Groups in Organic Synthesis" (Johne Wiley & Sons, New York, 1981) beschrieben. Salze von Verbindungen der Formel I mit salzbildenden Gruppen werden in an sich bekannter Weise hergestellt, indem man z.B eine Verbindung der Formel I mit einer basischen Gruppe mit einer stöchiometrischen Menge einer geeigneten Säure oder Verbindungen der Formel I mit einer sauren Gruppe mit einer stöchiometrischen Menge einer geeigneten Base umsetzt. Stereoisomerengemische, insbesondere Diastereomerengemische, die gegebenenfalls bei der Synthese von Verbindungen der Formel I anfallen, können in an sich bekannter Weise durch fraktionierte Kristallisation oder durch Chromatographie getrennt werden.

Die erfindungsgemäßen Verbindungen der Formel I weisen enzymhemmende Eigenschaften auf, insbesondere hemmen sie Aspartylproteasen wie das Renin.

Renin wird als Folge verschiedener Stimuli (Volumendepletion, Natriummangel, β-Rezeptorenstimulation) von den juxtaglomerulären Zellen der Niere in den Blutkreislauf sezerniert. Dort spaltet es von dem aus der Leber ausgeschiedenen Angiotensinogenen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkonstriktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotension II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, gereinigtes Humanrenin) gemessen.

**1. Testprinzip**

Z.B. Humanplasma, welches sowohl Renin als auch Angiotensinogen enthält, wird bei 37°C mit der zu testenden Verbindung inkubiert. Dabei wird aus Angiotensinogen unter der Einwirkung von Renin Angiotensin I freigesetzt, das anschließend mit einem handelsüblichen Radioimmunoassay gemessen werden kann. Diese Angiotensin-Freisetzung wird durch Renin-Inhibitoren gehemmt.

**2. Gewinnung des Plasmas**

Das Blut wird von freiwilligen Probanden gewonnen (ca. 0,5 l pro Person; Bluko-Entnahmegerät der Fa. ASID Bonz und Sohn, Unterschleißheim) und in teilweise evakuierten Flaschen unter Eiskühlung aufgefangen. Die Gerinnung wird durch Zugabe von EDTA (Endkonzentration 10 mM) verhindert. Nach dem zentrifugieren (Rotor HS 4 (Sorvall), 3 500 UpM, 0 - 4°C, 15 min.; wiederholen, falls erforderlich) wird das Plasma vorsichtig

abpipettiert und in geeigneten Portionen bei -30°C eingefroren. Für den Test werden nur Plasmen mit ausreichend hoher Reninaktivität verwendet. Plasmen mit niedriger Reninaktivität werden durch eine Kältebehandlung (-4°C, 3 Tage) aktiviert (Prorenin → Renin).

**3. Durchführung der Tests**

Angiotensin I wird mit dem Renin-Maia®-Kit (Serono Diagnostics S.A., Coinsins, Schweiz) bestimmt. Die Inkubation des Plasmas wird nach der dort angegebenen Anleitung durchgeführt:

| | |
|---|---|
| Inkubationsansatz: | 1000 µl Plasma (bei 0-4°C aufgetaut) |
| | 100 µl Phosphatpuffer (pH 7,4, Zusatz von $10^{-4}$ M Ramiprilat) |
| | 10 µl PMSF-Lösung |
| | 10 µl 0,1 % Genapol PFIC |
| | 12 µl DMSO bzw. Testpräparat |

Die Testpräparate werden i.a. $10^{-2}$ M in 100 % Dimethylsulfoxid (DMSO) gelöst und mit Wasser entsprechend verdünnt; der Inkubationsansatz enthält max. 1 % DMSO.

Die Ansätze werden in Eis gemischt und für 1 Stunde zur Inkubation in ein Wasserbad (37°C) gestellt. Aus einem zusätzlichen Ansatz ohne Inhibitor werden ohne weitere Inkubation insgesamt 6 Proben (jeweils 100 µl) zur Bestimmung des Ausgangs-Angiotensin I-Gehaltes des verwendeten Plasmas entnommen.

Die Konzentrationen der Testpräparate werden so gewählt, daß etwa der Bereich von 10 - 90 **%** Enzymhemmung abgedeckt ist (mindestens fünf Konzentrationen). Am Ende der Inkubationszeit werden aus jedem Ansatz drei 100 µl-Proben in vorgekühlten Eppendorf-Gefäßen auf Trockeneis eingefroren und bei ca. -25°C für die Angiotensin I-Bestimmung aufbewahrt (Mittelwert aus drei Einzelproben).

**4. Angiotensin I-Radioimmunoassay (RIA)**

Es wird exakt die Gebrauchsanweisung des RIA-Kits (Renin-Maia®-Kit, Serono Diagnostics S.A., Coinsins, Schweiz) befolgt.

Die Eichkurve umfaßt den Bereich von 0,2 bis 25,0 ng Angiotensin I pro ml. Der Basis-Angiotensin I-Gehalt des Plasmas wird von allen Meßwerten abgezogen. Die Plasma-Renin-Aktivität (PRA) wird als ng Ang I/ml x Stunde angegeben. PRA-Werte in Gegenwart der Testsubstanzen werden auf einen Ansatz ohne Inhibitor (= 100 %) bezogen und als % Restaktivität angegeben. Aus der Auftragung von % Reztaktivität gegen die Konzentration (M) des Testpräparates (logarithmische Skala) wird der $IC_{50}$-Wert abgelesen.

Die in der vorliegenden Erfindung beschriebenen Verbindungen der allgemeinen Formel I zeigen in dem in-vitro-Test Hemmwirkungen bei Konzentrationen von etwa $10^{-5}$ bis $10^{-10}$ Mol/l.

Im einzelnen wurden folgende Werte ermittelt:

Tabelle 1:

| Beispiel Nr. | $IC_{50}$ [M] humanes Plasmarenin | $IC_{50}$ [M] gereinigtes Humanrenin |
|---|---|---|
| 1 | $4,2 \cdot 10^{-7}$ | $1,2 \cdot 10^{-7}$ |
| 2 | $4,0 \cdot 10^{-9}$ | $4,0 \cdot 10^{-9}$ |
| 3 | $2,2 \cdot 10^{-8}$ | $1,2 \cdot 10^{-8}$ |
| 4 | $1,6 \cdot 10^{-9}$ | $2,4 \cdot 10^{-9}$ |
| 5 | $1,1 \cdot 10^{-8}$ | $1,4 \cdot 10^{-8}$ |
| 7 | $2,2 \cdot 10^{-7}$ | $3,0 \cdot 10^{-7}$ |
| 9 | $1,8 \cdot 10^{-8}$ | $1,2 \cdot 10^{-8}$ |
| 10 | $1,1 \cdot 10^{-7}$ | $5,0 \cdot 10^{-8}$ |
| 11 | $1,9 \cdot 10^{-6}$ | $3,6 \cdot 10^{-6}$ |
| 12 | $> 10^{-6}$ | $1,0 \cdot 10^{-5}$ |

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten, wie zum Beispiel Rhesus-Affen, herangezogen.

1. Testprinzip

Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen verabreicht und ihre Wirkung auf Blutdruck und Herzfrequenz wird gemessen.

2. Durchführung des Tests

6 Rhesusaffen wurden an 6 aufeinanderfolgenden Tagen mit 10 mg/kg·d Furosemid oral vorbehandelt. Am 7. Tag wurden etwa 30 Minuten vor Versuchsbeginn weitere 10 mg/kg Furosemid i.v. verabreicht. Anschließend wurde die Anästhesie mit 20 mg/kg Ketamin i.m. eingeleitet und mit 40 mg/kg Pentobarbiton i.v. fortgesetzt. Ein Seitenarm der Femoralarterie wurde freipräpariert und zur Blutdruckmessung mittels eines Blutdruck-Wandlers (P 23 ID) kanuliert. Blutproben zur Bestimmung der Plasma-Renin-Aktivität wurden über eine Braunüle in der Saphena-Vene abgenommen.

Die Titelverbindung des Beispiels 2 zeigt folgendes Ergebnis: 2 mg/kg i.d. (in 0,1 n Zitronensäure 2 mg/ml)

| t [min] | Blutdruck-Änderung [%] | Puls-Änderung [%] | Änderung PRA [%] |
|---|---|---|---|
| 1 | -0,72 | -1,16 | |
| 3 | -8,21 | -1,61 | |
| 5 | -11,53 | -2,42 | |
| 10 | -11,82 | -2,51 | |
| 15 | -12,68 | -3,49 | -20 |
| 20 | -13,48 | -1,97 | |
| 30 | -16,43 | -1,61 | -62 |
| 45 | -12,82 | +0,09 | -53 |
| 60 | -13,98 | -0,09 | -58 |
| 75 | - 8,79 | +1,16 | |
| 90 | - 6,77 | +3,04 | -51 |
| 120 | | | -47 |

Die Verbindungen der vorliegenden Erfindung sind hierbei in einem Dosisbereich von etwa 0,1 - 5 mg/kg i,v, wirksam, bei intraduonaler Applikation per Gastroskop im Dosisbereich von etwa 0,5 - 50 mg/kg.

Die in der vorliegenden Erfindung beschriebenen Verbindungen der allgemeinen Formel I können als Antihypertensiva sowie zur Behandlung der Herzinsuffizienz verwendet werden.

Zum Gegenstand der Erfindung gehört weiterhin die Verwendung von Verbindungen der Formel I zur Herstellung von Arzneimittel zur Bluthochdrucktherapie und der Behandlung der kongestiven Herzinsuffizienz.

Pharmazeutische Präparate enthalten eine wirksame Menge des Wirkstoffs der Formel I zusammen mit einem anorganischen oder organischen pharmazeutisch verwendbaren Trägerstoff.

Die Anwendung kann intranasal, intravenös, subkutan, peroral oder intraduodenal erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für die orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trockenals auch als Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.

Verzeichnis der verwendeten Abkürzungen:

| | |
|---|---|
| Ac | Acetyl |
| Boc | tert.-Butoxycarbonyl |
| BOP | Benzotriazol-1-yl-oxy-tris-(dimethylamino)-phosphonium |
| BuLi | n-Butyllithium |
| DC | Dünnschichtchromatographie |
| DCC | Dicyclohexylcarbodiimid |
| DCI | Desorption Chemical Ionisation |
| DIP | Diisopropylether |

| | |
|---|---|
| DNP | 2,4-Dinitrophenyl |
| DME | 1,2-Dimethoxyethan |
| DMF | Dimethylformamid |
| DMSO | Dimethylsulfoxid |
| EE | Essigsäureethylester |
| EI | Electron Impact |
| Etoc | Ethoxycarbonyl |
| FAB | Fast atom bombardment |
| H | Hexan |
| Hep | n-Heptan |
| HOBt | 1-Hydroxybenzotriazol |
| Iva | Isovaleryl |
| M | Molekularpeak |
| MeOH | Methanol |
| MS | Massenspektrum |
| MTB | Methyl-tert.-butylether |
| NEM | N-Ethylmorpholin |
| Nva | Norvalin |
| Nle | Norleucin |
| PPA | n-propanphosphonsäureanhydrid |
| R.T. | Raumtemperatur |
| Schmp. | Schmelzpunkt |
| $Sdp_{xx}$ | Siedepunkt bei xx Torr |
| TBTU | 2-(1H-Benzotriazol-1-yl)-1,1,3,3-tetramethyluronium |
| Thi | 2-Thienylalanin |
| THF | Tetrahydrofuran |
| Z | Benzyloxycarbonyl. |

Kürzelliste ausgewählter C-Termini

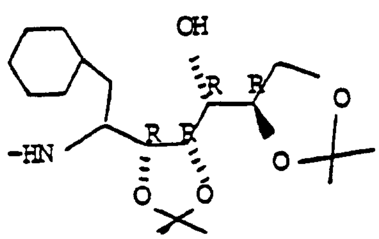

(D)-Manno-Pentol (XX)

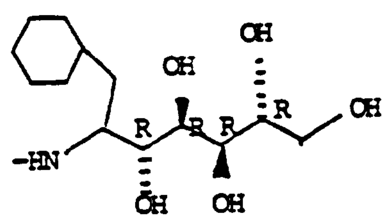

(D)-Manno-Pentol

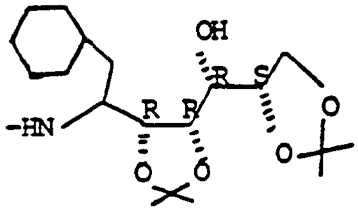

(L)-Gulo-Pentol (XX)

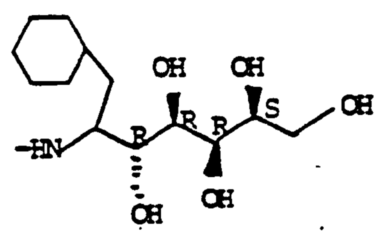

(L)-Gulo-Pentol

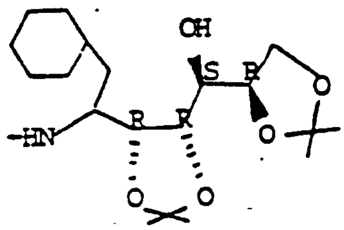

(D)-Talo-Pentol(XX)

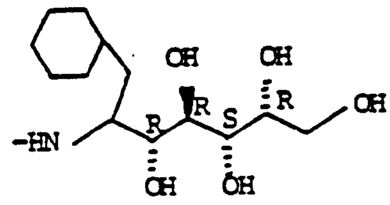

(D)-Talo-Pentol

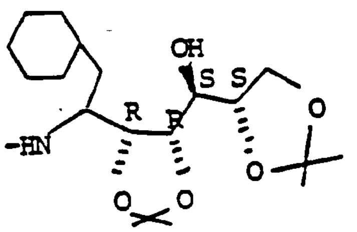

(L)-Allo-Pentol(XX)

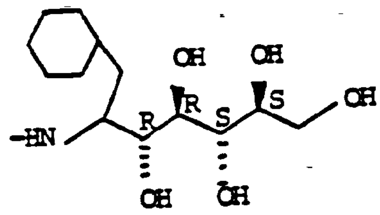

(L)-Allo-Pentol

Die sonstigen für Aminosäuren verwendeten Abkürzungen entsprechen dem in der Peptidchemie üblichen Drei-Buchstaben-Code wie er z.B. in Eur. J. Biochem. 138, 9 - 37 (1984) beschrieben ist. Falls nicht ausdrücklich anders angegeben, handelt es sich immer um Aminosäuren der L-Konfiguration.

Die nachstehenden Beispiele dienen zur Erläuterung der vorliegenden Erfindung, ohne daß diese darauf beschränkt wäre.

**Beispiel 1**

Iva-Phe-Nva-[(L)-Gulo-Pentol]

200 mg Iva-phe-Nva-[(L)-Gulo-Pentol(XX)] werden in 10 ml 85 % wäßriger Trifluoressigsäure gelöst und 2,5 Stunden bei R.T. gerührt. Die Solventien werden i.Vak. entfernt und an Kieselgel mit $CH_2Cl_2$/MeOH 10:1 chromatographiert. Man erhält 140 mg der Titelverbindung als farbloses amorphes Pulver.
$R_f$ ($CH_2Cl_2$/MeOH 10:1) = 0,06 MS (FAB + LiJ): 614 (M+Li)

a) Iva-Phe-Nva-[(L)-Gulo-Pentol(XX)]

578 mg Iva-Phe-Nva-OH, 229 µl Ethylpiperidin und 230 µl Triethylamin werden in 25 ml wasserfreiem $CH_2Cl_2$ gelöst und bei -15°C mit 204 µl Pivaloylchlorid versetzt. 10 Minuten wird bei R.T. gerührt, auf -10°C gekühlt und mit einer Lösung von 599 mg H-(L)-Gulo-Pentol(XX) in 10 ml wasserfreiem $CH_2Cl_2$ versetzt. 20 Stunden wird bei R.T. gerührt, das Solvens i.Vak. entfernt und in 150 ml EE aufgenommen. Dreimal wird mit je 50 ml $KH_2PO_4$-Lösung und dreimal mit je 50 ml $NaHCO_3$-Lösung gewaschen, die EE-Phase über $K_2CO_3$ getrocknet und das Solvens i.Vak. entfernt. Chromatographie an Kieselgel mit DIP/MTB 1:1 liefert 200 mg der Titelverbindung als farbloses amorphes Pulver.
$R_f$ (MTB/DIP 1:1) = 0,10 MS(FAB): 688 (M+1)

b) [H-(L)-Gulo-Pentol(XX)]

740 mg [N-Benzyl-(L)-Gulo-Pentol(XX)] werden in 20 ml wasserfreiem MeOH gelöst und unter Argon mit 150 mg Pd/C (10 %) und 1,1 g $HCO_2NH_4$ versetzt. 1,5 Stunden wird unter Rückfluß erhitzt, vom Katalysator abfiltriert und das Solvens i.Vak. entfernt. Man erhält 600 mg der Titelverbindung als blaßgelbes Öl, das ohne Reinigung weiter eingesetzt wird.
$R_f$ (MTB) = 0,05

c) [N-Benzyl-(L)-Gulo-Pentol(XX)]

880 mg 2-Benzylamino, 2-Cyclohexylmethyl, (3,4-Isopropyliden)-3(S),4(S)-dihydroxy, 5-(R)-[(1,2-isopropyliden)-1(S), 2-dihydroxyethyl]-tetrahydrofuran werden in 25 ml wasserfreiem THF gelöst und mit 188 mg $LiAlH_4$ versetzt. 20 Stunden wird bei R.T. gerührt, mit 50 ml $NaHCO_3$-Lösung versetzt und dreimal mit je 100 ml EE extrahiert. Über $Na_2SO_4$ wird getrocknet und das Solvens i.Vak. entfernt. Man erhält 690 mg der Titelverbindung als farbloses Öl.
$R_f$ (MTB/Hep 1:5) = 0,31 MS (DCl): 448 (M+1)

d) 2-Benzylamino,2-Cyclohexylmethyl,(3,4-isopropyliden)-3(S),4(S)-dihydroxy,5-(R)-[(1,2-isopropyliden)-1(S),2-dihydroxyethyl]-tetrahydrofuran

4,65 g 2-Cyclohexylmethyl,(3,4-isopropyliden)-2,3(S), 4(S)-trihydroxy,5-(R)-[(1,2-isopropyliden)-1(S),2-dihydroxyethyl]-tetrahydrofuran und 5,7 ml Benzylamin werden in 150 ml wasserfreiem Toluol gelöst und bei -20°C mit 910 µl $TiCl_4$ versetzt. Man rührt 3 Stunden bei R.T., versetzt mit 100 ml gesättigter wäßriger $Na_2CO_3$-Lösung, verdünnt mit 300 ml EE und wäscht mit $KH_2PO_4$-Lösung bis pH 5 erreicht ist. Die organische Phase wird mit $Na_2SO_4$ getrocknet und das Solvens i. Vak. entfernt.
Chromatographie an Kieselgel mit MTB/Hep 1:5 liefert 1,0 g der Titelverbindung als farbloses Öl.
$R_f$ (MTB/Hep 1:5) = 0,24 MS (DCl): 446 (M+1)

e) 2-Cyclohexylmethyl,(3,4-isopropyliden)-2,3(S),4(S)-trihydroxy,5-(R)-[(1,2-isopropyliden)-1(S),2-dihydroxyethyl]-tetrahydrofuran

5,16 g (2,3-, 5,6-Diisopropyliden)-L-Gulonsäure-γ-lacton wird in 300 ml Diethylether suspendiert und unter Argon bei Rückflußtemperatur 1,1 Äquivalente Cyclohexylmethyl-magnesiumbromid in 50 ml Diethylether im Verlauf von 2 Stunden zugetropft. Anschließend wird mit 100 ml gesättigter wäßriger $NaHCO_3$-Lösung versetzt, zweimal mit je 100 ml EE extrahiert, die organische Phase über $Na_2SO_4$ getrocknet und das Solvens i.Vak. entfernt. Man erhält 4,9 g der Titelverbindung als farbloses Öl, leicht verunreinigt mit Bisaddukt.
$R_f$ (DIP) = 0,40 MS (DCl): 357 (M + 1)

f) (2,3-,5,6-Diisopropyliden)-L-Gulonsäure-γ-Lacton

42 g L-Gulonsäure-γ-lacton und 100 mg p-Toluolsulfonsäure werden in 300 ml 2,2-Dimethoxypropan 5 Stunden unter Rückfluß erhitzt. Nach 1 Stunde ensteht eine klare Lösung. Die flüchtigen Bestandteile werden i. Vak. entfernt und aus DIP umkristallisiert. Man erhält 45 g der Titelverbindung als farblose Kristalle, Schmp.: 144°C.
$R_f$ (DIP) = 0,12 MS (DCl): 259 (M+1)

**Beispiel 2**

(2(S)-Benzyl,3-t-butylsulfonyl)propionyl-His-[(D)-Manno-Pentol]

960 mg (2(S)-Benzyl,3-t-butylsulfonyl)propionyl-His-[(D)-Manno-Pentol(XX)] werden mit 722 mg p-Toluolsulfonsäure in 100 ml Methanol gelöst und 2 Tropfen Wasser zugegeben. Man rührt 17 Stunden bei RT, dann wird mit $NaHCO_3$-Lösung auf pH = 7 gestellt, das Methanol im Vakuum entfernt, dreimal mit 100 ml MTB extrahiert und über $Na_2SO_4$ getrocknet. Das Solvens wird im Vakuum entfernt und der Rückstand an Kieselgel mit Aceton/Wasser 10:1 chromatographiert. Man erhält 120 mg der Titelverbindung als weiße Kristalle.
$R_f$ (Aceton/Wasser 10:1) = 0,46 MS (FAB): 681 (M+1)
Schmp. 100 - 110°C (Zersetzung)

a) (2(S)-Benzyl,3-t-butylsulfonyl)propionyl-His-[(D)-Manno-Pentol(XX)]

850 mg (2(S)-Benzyl,3-t-butylsulfonyl)propionyl-His(DNP)-[(D)-Manno-Pentol(XX)] und 0,94 ml Thiophenol werden in 15 ml Acetonitril gelöst und 2 Stunden bei RT gerührt. Das Solvens wird im Vakuum entfernt, der Rückstand mit DIP digeriert und der Rückstand an Kieselgel mit Toluol/MeOH 5:1 chromatographiert. Man erhält 400 mg der Titelverbindung als blaßgelbe Kristalle.
Schmp. ≈ 160°C (Zersetzung)
$R_f$ (Toluol/MeOH 5:1) = 0,39 MS (FAB): 761 (M+1)

b) (2(S)-Benzyl,3-t-butylsulfonyl)propionyl-His(DNP)-[(D)-Manno-Pentol(XX)]

1,3 g (2(S)-Benzyl,3-t-butylsulfonyl)propionyl-His(DNP)-OH, 0,31 ml N-Ethylpiperidin und 0,31 ml Triethylamin werden in 30 ml $CH_2Cl_2$ gelöst und bei -15°C unter Argon 0,28 ml Pivaloylchlorid zugetropft. 10 Minuten wird bei RT gerührt, auf -10°C gekühlt und eine Lösung von 400 mg H-(D)-Manno-Pentol(XX) in 5 ml $CH_2Cl_2$ zugetropft. 18 Stunden wird bei RT gerührt, mit 100 ml MTB verdünnt und je einmal mit 100 ml 0,66 M $KH_2PO_4$ und 100 ml gesättigter $NaHCO_3$-Lösung extrahiert. Über $Na_2SO_4$ wird getrocknet und das Solvens im Vakuum entfernt. Man erhält 850 mg der Titelverbindung als amorphes Pulver, das ohne Reinigung weiter umgesetzt wird.
$R_f$ (EE/MeOH 10:1) = 0,53 MS (FAB): 927 (M+1)

c) (2(S)-Benzyl,3-t-butylsulfonyl)propionyl-His(DNP)-OH

10,7 g (2(S)-Benzyl,3-t-benzylsulfonyl)propionsäure-N-hydroxy-succinimidester und 11,3 g H-His(DNP)-OH-hydrochlorid werden in 500 ml THF/Ethanol 1:1 gelöst und mit 470 ml gesättigter wäßriger $NaHCO_3$-Lösung versetzt. 5 Stunden wird bei RT gerührt, die organischen Solventien im Vakuum entfernt und mit $NaHSO_4$-Lösung auf pH = 1-2 gestellt, dreimal wird mit je 500 ml EE extrahiert, über $Na_2SO_4$ getrocknet und das Solvens im Vakuum entfernt. In 350 ml Aceton/EE 1:1 wird aufgenommen und im Ultraschallbad mit Diethylether ausgefällt. Man erhält 12,5 g der Titelverbindung als gelbes Pulver.
$R_f$ (MeOH/$CH_2Cl_2$ 1:5) = 0,11 MS (FAB): 588 (M+1)

d) (2(S)-Benzyl,3-t-butylsulfonyl)propionsäure-N-hydroxy-succinimidester

8,0 g (2(S)-Benzyl,3-t-butylsulfonyl)propionsäure (J. Med. Chem. 31, 1839 (1988)) und 3,3 g N-Hydroxysuccinimid werden in 200 ml 1,2-Dimethoxyethan gelöst und bei 0°C eine Lösung von 5,8 g DCC in 50 ml 1,2-Dimethoxyethan zugetropft. 18 Stunden wird bei 6°C stehen gelassen, bei 20°C das Solvens im Vakuum entfernt und in 100 ml Acetonitril aufgenommen. Vom Harnstoff wird abfiltriert und das Solvens bei 20°C im Vakuum entfernt. Man erhält 10,7 g der Titelverbindung als farbloses Öl, das ohne Reinigung weiter umgesetzt wird.

e) H-(D)-Manno-Pentol(XX)

Die Verbindung wird analog Beispiel 1b aus N-Benzyl-(D)- Manno-pentol(XX) hergestellt.

f) [N-Benzyl-(D)-Manno-Pentol(XX)]

8,8 g 2-Benzylamino,(3,4-isopropyliden)-3(S),4(S)-dihydroxy,5(R)-[(1,2-isopropyliden)-1(R),2-dihydroxyethyl]-tetrahydrofuran und 7,1 ml Cyclohexylmethylbromid werden in 250 ml THF gelöst und mit 0,7 g Lithiumdraht (3,2 mm ϕ, ca. 1 % Na) unter Argon im Ultraschallbad bei RT umgesetzt. Nach 6 Stunden wird das Solvens im Vakuum entfernt, mit 500 ml 0,67 M $KH_2PO_4$-Lösung aufgenommen und dreimal mit 300 ml MTB extrahiert. Über $Na_2SO_4$ wird getrocknet, das Solvens im Vakuum entfernt und der Rückstand an Kieselgel mit MTB/Hep 1:1 chromatographiert. Man erhält 3,3 g der Titelverbindung als farbloses Öl.
$R_f$ (MTB/Hep 1:1) = 0,39 MS(DCI): 448 (M+1)

g) 2-Benzylamino,(3,4-isopropyliden)-3(S),4(S)-dihydroxy, 5(R)-[(1,2-isopropyliden)-1(R),2-dihydroxyethyl]-tetrahydrofuran

31,5 g 2,3-,5,6-Diisopropyliden-D-mannofuranosid und 20,0 ml Benzylamin werden in 250 ml Toluol am Wasserabscheider erhitzt. Nach 7 Stunden wird der Wasserabscheider durch einen mit Molekularsieb 4Å gefüllten Soxhlet-Extrator ersetzt und weitere 16 Stunden unter Rückfluß erhitzt. Anschließend wird das Solvens im Vakuum entfernt, mit 500 ml EE aufgenommen und dreimal mit 250 ml 0,67 M $KH_2PO_4$-Lösung gewaschen. Über $Na_2SO_4$ wird getrocknet und das Solvens im Vakuum entfernt. Man erhält 41,5 g der weißen, kristallinen Titelverbindung.
$R_f$ (Toluol/EE 2:1) = 0,29 MS(DCI): 350 (M+1)

**Alternativsynthesen**

h) (2(S)-Benzyl,3-t-butylsulfonyl)-propionyl-His-[(D)-Manno-Pentol(XX)] (2a))

785 mg (2(S)-Benzyl,3-t-butylsulfonyl)-propionsäure und 382 µl Triethylamin werden in 20 ml DMF gelöst und bei RT 1,1 g O-Benzotriazol-1-yl-1,1,3,3-tetramethyluronium-hexafluorphosphat zugefügt und 5 min. bei RT gerührt. Dann wird eine Lösung von 1,6 g H-His-[(D)-Manno-Pentol(XX)] in 15 ml DMF zugetropft und 20 h bei RT gerührt. Das Solvens wird i. Vak. entfernt, mit 500 ml EE aufgenommen und dreimal mit 100 ml $Na_2CO_3$-Lösung gewaschen. Über $Na_2SO_4$ wird getrocknet, das Solvens i.Vak. entfernt und an Kieselgel mit EE/MeOH 5:1 chromatographiert. Man erhält 1,1 g der Titelverbindung des Beispiels 2a).

i) H-His-[(D)-Manno-Pentol(XX)]

3 g Z-His-[(D)-Manno-Pentol(XX)] und 3 g Ammoniumformiat werden in 50 ml Methanol gelöst, 2 g Pd/C (10 %) zugefügt und 5 h unter Argon bei R.T. gerührt. Dann wird der Katalysator abfiltriert, das Solvens i.Vak. entfernt, in 500 ml EE aufgenommen und dreimal mit 50 ml $Na_2CO_3$-Lösung gewaschen. Über $Na_2SO_4$ wird getrocknet und das Solvens i.Vak. entfernt. Man erhält 1,8 g der Titelverbindung als Schaum, der unter Argon aufbewahrt und möglichst schnell weiter umgesetzt wird.

k) Z-His-[(D)-Manno-Pentol(XX)]

500 mg H-(D)-Manno-Pentol(XX) und 405 mg Z-His-OH werden unter Argon in 20 ml DMF gelöst, anschließend bei 0°C zunächst mit 303 µl Diphenylphosphorylazid, dann mit 208 µl 1-Diethylamino-2-propanol versetzt.Man rührt 2 h bei 0°C und anschließend 4 d bei RT. Das Reaktionsgemisch wird mit 200 ml EE verdünnt und je einmal mit 100 ml 0,7 M $KH_2PO_4$-Lösung und 100 ml gesättigter $NaHCO_3$-Lösung gewaschen. Über $Na_2SO_4$ wird getrocknet und an Kieselgel mit EE/MeOH 10:1 chromatographiert. Man erhält 530 mg der Titelverbindung, farbloser Schaum.
$R_f$ (EE/MeOH 10:1) = 0,17 MS (FAB): 629 (M+1)

1) H-(D)-Manno-Pentol(XX)

410 mg N-Benzhydryl-(D)-Manno-Pentol(XX) und 490 mg Ammoniumformiat werden in 15 ml wasserfreiem Methanol gelöst, mit 82 mg Pd/C (10 %) versetzt und unter Argon 6 h bei RT gerührt. Das Solvens wird i.Vak. entfernt, in 100 ml EE aufgenommen und dreimal mit 50 ml $Na_2CO_3$-Lösung gewaschen. Die organische Phase wird mit $Na_2SO_4$ getrocknet und das Solvens i.Vak. entfernt. Nach Feinvakuum-Trocknung zur Entfernung des Diphenylmethans erhält man 275 mg der Titelverbindung des Beispiels 2e).

m) N-Benzhydryl-(D)-Manno-Pentol(XX)

1,8 g 2-Benzhydrylamino,(3,4-isopropyliden)-3(S),4(S)-dihydroxy,5(R)-[1,2-isopropyliden)-1(R),2-dihydroxyethyl]-tetrahydrofuran und 1,2 ml Cyclohexylmethylbromid werden in 40 ml Formaldehyddimethylacetal (von K/Na-Legierung destilliert) gelöst und mit 115 mg Lithiumdraht (3,2 mm ϕ, ca. 1 % Na) unter Argon im Ultraschallbad bei 20 - 40°C 3,5 h umgesetzt. Dann wird erneut 115 mg Lithiumdraht und 1,2 ml Cyclohexylmethylbromid zugesetzt und noch 1 h bei 40 - 60°C umgesetzt. Man gießt das Reaktionsgemisch in 200 ml $NaHCO_3$-Lösung und extrahiert dreimal mit 100 ml MTB. Über $Na_2SO_4$ wird getrocknet, das Solvens i.Vak. entfernt und an Kieselgel mit DIP/Toluol 1:3 chromatographiert. Man erhält 1,1 g der Titelverbindung als farbloses Öl.
$R_f$ (DIP/Toluol 1:3) = 0,28 MS(DCI): 524 (M+1)

n) 2-Benzhydrylamino,(3,4-isopropyliden)-3(S),4(S)-dihydroxy, 5(R)-[1,2-isopropyliden-1(R),2-dihydroxy-ethyl]-tetrahydrofuran

10 g D(+)-Mannose und etwa 10 mg p-Toluolsulfonsäure werden in 40 ml 2,2-Dimethoxypropan suspendiert und bei 40°C 1 h gerührt. Dabei entsteht eine klare Lösung. 10 ml Benzhydrylamin wird zugegeben und 24 h am Rückfluß gekocht. Dann werden weitere 10 ml 2,2-Dimethoxypropan und 2 ml Benzhydrylamin zugegeben und weitere 18 h am Rückfluß gekocht. Flüchtige Bestandteile werden i.Vak. entfernt, in 100 ml EE aufgenommen und dreimal mit 100 ml $NaHCO_3$-Lösung gewaschen. Über $Na_2SO_4$ wird getrocknet, das Solvens i.Vak. entfernt und an Kieselgel mit DIP/Toluol 1:5 chromatographiert. Man erhält 17 g der Titelverbindung als blaßgelbe Kristalle, Fp: 82-84°C.

$R_f$(DIP/Toluol 1:3) = 0,41 MS(DCI): 426 (M+1)

Analog kann auch die Titelverbidung des Beispiels 2g) im Eintopfverfahren aus D(+)-Mannose hergestellt werden.

**Beispiel 3**

cis-4-Aminocyclohexylcarbonyl-Phe-Bis-[(D)-Manno-Pentol]

180 mg (N-tert.-butoxycarbonyl-cis-4-amino-cyclohexylcarbonyl)-Phe-His-[(D)-Manno-Pentol(XX)] werden in 5 ml $CH_2Cl_2$ gelöst und bei 0°C 5 ml Trifluoressigsäure addiert. 24 Stunden wird bei RT gerührt, 100 ml gesättigte $Na_2CO_3$-Lösung zugegeben und 3x mit 100 ml EE extrhiert. Über $Na_2SO_4$ wird getrocknet, das Solvens im Vakuum entfernt und der Rückstand an Kieselgel mit Aceton/$H_2O$/konz. $NH_3$ 100:10:5 chromatographiert. Man erhält 24 mg der Titelverbindung als farbloses Pulver.
$R_f$ (Aceton/$H_2O$/konz. $NH_3$ 100:10:5) = 0,12
MS(FAB): 687 (M+1)

a) (N-Tert.-butoxycarbonyl-cis-4-aminocyclohexylcarbonyl)-Phe-His-[(D)-Manno-Pentol(XX)]

Die Titelverbindung wird analog Beispiel 2a), 2b) aus (N-Tert.-butoxycarbonyl-cis-4-aminocyclohexylcarbonyl)-Phe-His(DNP)-OH und H-(D)-Manno-Pentol(XX) hergestellt.
$R_f$ (EE/MeOH 5:1) = 0,43 MS(FAB): 868 (M+1)

b) (N-Tert.-butoxycarbonyl-cis-4-aminocyclohexylcarbonyl)-Phe-His(DNP)-OH

Die Titelverbindung wird analog Beispiel 2c), 2d) aus (N-Tert.-butoxycarbonyl-cis-4-aminocyclohexylcarbonyl)-Phe-OH und H-His(DNP)-OH hergestellt.
$R_f$ (EE/MeOH 3:1) = 0,20 MS(FAB): 694 (M+1)

c) N-(N-Tert.-butoxycarbonyl-cis-4-aminocyclohexylcarbonyl)-L-phenylalanin

5,5 g N-(N-Tert.-butoxycarbonyl-cis-4-aminocyclohexylcarbonyl)-L-phenylalaninbenzylester werden in 230 ml Ethanol über 1 g Pd/Kohle unter Normaldruck hydriert. Nach beendeter Reaktion wurde vom Katalysator abfiltriert und das Lösungsmittel abdestilliert. Nach Umkristallisation aus n-Heptan/Essigester werden 4,1 g farbloses Produkt erhalten.
Schmelzpunkt 160 - 161°C (Zersetzung)
MS(DCI): 391 (M+1)

d) N-(N-Tert.-butoxycarbonyl-cis-4-aminocyclohexylcarbonyl)-L-phenylalaninbenzylester

6,0 g N-Tert.-butoxycarbonyl-cis-1,4-aminocyclohexancarbonsäure und 6,3 g L-Phenylalaninbenzylester werden in 75 ml DMF gelöst und mit 24 ml n-PPA und 15,7 ml NEM bei 0°C gemischt und über Nacht bei RT zur Reaktion gebracht. Die Lösung wird mit $CH_2Cl_2$ verdünnt und jeweils mit halbgesättigter $NaHCO_3$-Lösung, 10 %-Citronensäure und Wasser gewaschen, über $MgSO_4$ getrocknet und im Vakuum eingeengt. Flashchromatographie ergab 5,7 g reines Produkt.
$[\alpha]_D^{20}$: -14,6° (c = 1,1 in $CH_3OH$)

**Beispiel 4**

2(S)-{N-Methyl-N-<2-[N-(morpholinocarbonyl)-N-methylamino]-ethyl>-aminocarbonyloxy}-3-phenylpropionyl-His-[(D)-Manno-Pentol]

Die Titelverbindung wird analog Beispiel 2a), 2b), 2c) und 2d) aus 2(S)-{N-Methyl-N-<2-[N-(morpholinocarbonyl)-N-methylamino]ethyl>-aminocarbonyloxy}-3-phenylpropionsäure hergestellt.
$R_f$ (EE/MeOH 1:1) = 0,22 MS(FAB): 790 (M+1)

a) 2(S)-{N-Methyl-N-<2-[N-(morpholinocarbonyl)-N-methylamino]ethyl>-aminocarbonyloxy}-3-phenylpropionsäure

840 g 2(S)-{N-Methyl-N-<2-[N-(morpholinocarbonyl)-N-methylamino]ethyl>-aminocarbonyloxy}-3-phenylpropionsäure-ethylester werden in 100 ml Methanol gelöst und mit 20 ml 0,1 n Natriumhydroxyd-Lösung versetzt. Nach 16 Stunden bei RT wird das Methanol abdestilliert und der Rückstand mit Salzsäure auf pH 1 - 2 eingestellt. 3-maliges Extrahieren mit EE liefert nach Trocknen mit $Na_2SO_4$ und Einengen die Titelverbindung (24 %), die ohne weitere Reinigung für die nächsten Reaktionen verwendet wird.
MS (DCI): 394 (M+1)

b) 2(S)-{N-Methyl-N-<2-[N-(morpholinocarbonyl)-N-methylamino]ethyl>-aminocarbonyloxy}-3-phenylpropionsäure-ethylester

Zu 2 g Di(4-benzotriazolyl)carbonat und 0,23 ml Pyridin in $CH_2Cl_2$ (20 ml) werden bei RT 0,9 g Phenylmilchsäure-ethylester in $CH_2Cl_2$ (10 ml) zugetropft. Nach 6 Stunden werden 900 mg N-Methyl-N-2-[N-(morpholinocarbonyl)-N-methylamino]-ethylamin in 10 ml $CH_2Cl_2$ zugetropft. Nach 16 Stunden werden 100 ml Ethylacetat zugegeben und anschließend zweimal mit gesättigter $Na_2CO_3$-Lösung, zweimal mit gesättigter $NaHSO_4$ und 1x mit gesättiger NaCl-Lösung gewaschen. Nach Trocknen mit $Na_2SO_4$ und Einengen erhält man ein gelbes Öl, das nach Chromatographie an $SiO_2$ (Eluens EE) die Titelverbindung liefert.
$R_f$ (EE) = 0,3 MS (DCI): 422 (M+1)

c) N-Methyl-N-2-[N-(morpholinocarbonyl)-N-methylamino]-ethylamin

2,1 g Boc-N-Methyl-N-2-[N-(morpholinocarbonyl)-N-methylamino]ethylamin werden in 75 ml ~6N Lösung von HCl in DME 3 Stunden bei RT gerührt. Nach Einengen werden 50 ml gesättigte $Na_2CO_3$-Lösung zugegeben und dreimal mit EE extrahiert. Nach Trocknen und Einengen erhält man die Titelverbindung, die ohne weitere Reinigung für die weiteren Reaktionen eingesetzt wird.

d) Boc-N-methyl-N-2-[N-(morpholinocarbonyl)-N-methylamino]-ethylamin

Die Titelverbindung wird analog Beispiel 4b) aus 2,1 g Morpholin, 10 g Di-(1-benzotriazolyl)carbonat, 2 ml Pyridin und 4,7 g Boc-N-methyl-2-(methylamino)ethylamin erhalten.
$R_f$ (EE) = 0,25 MS (DCI): 302 (M+1)

e) Boc-N-methyl-2-(methylamino)ethylamin

Zu 100 g N,N'-Dimethylethylendiamin werden bei 5°C 12,5 g Di-t-butyldicarbonat in 25 ml $CH_2Cl_2$ zugetropft. Nach 4 Stunden bei RT wird das überschüssige Diamin abdestilliert. Der Rückstand wird in EE (100 ml) aufgenommen und mit gesättigter $Na_2CO_3$-Lösung und gesättigter NaCl-Lösung gewaschen. Nach Trocknen mit $Na_2SO_4$ und Einengen erhält man die Titelverbindung, die roh für weitere Reaktionen verwendet wird.
MS (DCI): 189 (M+1)

**Beispiel 5**

3-(4-Amino-1-piperidinyl-carbonyl)-2(R)-benzylpropionyl-His-[(D)-Manno-Pentol]

Die Titelverbindung wird analog Beispiel 3 aus 3-[4-(tert.-Butoxycarbonyl)amino-1-piperidinyl-carbonyl]-2(R)-benzylpropionsäure hergestellt.
$R_f$ (Aceton/$H_2O$/konz. $NH_3$ 100:10:5) = 0,15
MS (FAB): 687 (M+1)

a) 3-[4-(tert.-Butoxycarbonyl)amino-1-piperidinyl-carbonyl]-2(R)-benzylpropionsäure

1,3 g 3-[4-(tert.-Butoxycarbonyl)amino-1-piperidinyl-carbonyl]-2(R)-benzylpropionsäure-benzylester werden in 60 ml EtOH mit 200 mg Pd/C 1 Stunde lang bei RT hydriert. Nach Filtrieren und Entfernung des Solvens im Vakuum kristallisiert die Titelverbindung aus kaltem Diethylether aus.
Schmp.: 135 - 136°C
$R_f$ ($CH_2Cl_2$/MeOH 9:1) = 0,30 MS (DCI): 391 (M+1)

b) 3-[4-(tert.-Butoxycarbonyl)amino-1-piperidinyl-carbonyl]-2(R)-benzylpropionsäure-benzylester

1,0 g 2(R)-(Carboxymethyl)-3-phenyl-propionsäure-benzyl-ester (J. Med. Chem. 31 2277 (1988)) werden in 50 ml $CH_3Cl_2$ bei 0°C mit 0,31 ml Oxalylchlorid und 1 ml DMF 1 Stunde lang gerührt. Das Solvens wird im Vakuum entfernt, in 25 ml $CH_2Cl_2$ aufgenommen, mit 2 Tropfen Triethylamin auf pH = 7 gestellt und zu dieser Lösung 0,71 g 4-(tert.-Butoxycarbonyl)-amino-piperidin und 0,47 ml Triethylamin in 50 ml $CH_2Cl_2$ zugetropft, 3 Stunden wird bei 0°C gerührt, das Solvens im Vakuum entfernt, in 50 ml EE aufgenommen und je einmal mit 50 ml 2N HCl und gesättigter $NaHCO_3$-Lösung gewaschen. Über $MgSO_4$ wird getrocknet, das Solvens im Vakuum entfernt und man erhält 1,3 g der Titelverbindung als farbloses Öl.
$R_f$ ($CH_2Cl_2$/MeOH 9:1) = 0,50 MS (DCI): 479 (M+H)

c) 4-(tert.-Butoxycarbonyl)amino-piperidin

10,0 g 1-Benzyl-4-[(tert.-butoxycarbonyl)amino]-piperidin werden in 60 ml Ethanol/Eisessig 9:1 mit 1 g Pd/C l Stunde bei RT und 1 bar Druck hydriert. Der Katalysator wird abfiltriert, das Solvens im Vakuum entfernt (Reste Eisessig werden azeotrop mit Toluol abdestilliert) und in 100 ml EE aufgenommen. Anschließend wird je einmal mit 100 ml gesättigter $NaHCO_3$-Lösung und gesättigter NaCl-Lösung gewaschen, über $MgSO_4$ getrocknet und das Solvens im Vakuum entfernt. Der Rückstand wird aus EE umkristallisiert und man erhält 5,5 g der Titelverbindung als weiße Kristalle.
Schmp.: 159 - 161°C
$R_f$ ($CH_2Cl_2$/MeOH 9:1) = 0,11 MS (DCI): 201 (M+1)

d) 1-Benzyl-4-[(tert.-butoxycarbonyl)amino]-piperidin

10,0 g 4-Amino-N-benzylpiperidin werden in 100 ml $CH_2Cl_2$ gelöst und mit 11,5 g Di-tert.-butyldicarbonat versetzt, die Lösung 2 Stunden lang bei RT gerührt und über Nacht stehen gelassen. Das Solvens wird im Vakuum entfernt, und aus EE umkristallisiert. Man erhält 12,9 g der Titelverbindung als weiße Kristalle.
Schmp.: 123°C
$R_f$ ($CH_2Cl_2$/MeOH 8:1) = 0,23 MS (DCI): 291 (M+1)

**Beispiel 6**

2(S)-(4-Amino-1-piperidinocarbonyloxy)-3-phenylpropionyl-His-[(D)-Manno-Pentol]

Die Titelverbindung wird analog Beispiel 3 aus 2(S)-[4-(tert.-Butoxycarbonyl)amino-1-piperidinocarbonyloxy]-3-phenylpropionsäure hergestellt
$R_f$ (Aceton/$H_2O$/konz. $NH_3$ 100:10:5) = 0,15
MS (FAB): 689 (M+1)

a) 2(S)-[4-(tert.-Butoxycarbonyl)amino-1-piperidinocarbonyloxy]-3-phenylpropionsäure

1,8 g (2(S)-[4-(tert.-Butoxycarbonyl)amino-1-piperidinocarbonyloxy]-3-phenylpropionsäure-ethylester und 5,1 ml 1N NaOH werden in 30 ml Ethanol gelöst und 18 Stunden bei RT gerührt. Anschließend wird mit 50 ml $H_2O$ verdünnt, Ethanol im Vakuum entfernt und mit $NaHSO_4$-Lösung auf pH = 1 - 2 gestellt. Dreimal wird mit 100 ml EE extrahiert, über $Na_2SO_4$ getrocknet und das Solvens im Vakuum entfernt. Man erhält die Titelverbindung, die aus Ether/n-Heptan kristallisiert, 1,0 g weiße Kristalle.
Schmp.: 100 - 101°C
$R_f$ ($CH_2Cl_2$/MeOH) = 0,29 MS (DCI): 393 (M+1)

b) 2(S)-[4-(tert.-Butoxycarbonyl)amino-1-piperidinocarbonyloxy]-3-phenylpropionsäure-ethylester

825 mg Phenylmilchsäureethylester und 1,8 g Di-(1-benzotriazolyl)-carbonat (70.%) werden in 40 ml $CH_2Cl_2$ gelöst 386 µl Ethyldiisopropylamin addiert und bei RT 18 Stunden gerührt. Dann wird 850 mg 4-(tert.-Butoxycarbonyl)aminopiperidin (Beispiel 5c)), gelöst in 10 ml $CH_2Cl_2$, zugetropft und weitere 386 µl Ethyldiisopropylamin addiert. Weitere 2 Stunden wird bei RT gerührt, das Solvens im Vakuum entfernt und mit 100 ml MTB aufgenommen. Mit je 100 ml $Na_2CO_3$-Lösung und 100 ml $NaHSO_4$-Lösung wird gewaschen, über $Na_2SO_4$ getrocknet und das Solvens im Vakuum entfernt. Man erhält 1,8 g der Titelverbindung als farbloses Öl, das ohne Reinigung weiter eingesetzt wird.

$R_f$ (Hep/EE 2:1) = 0,2 MS (DCI): 421 (M+1)

**Beispiel 7**

2(S)-(4-Aminomethyl-1-piperidinocarbonyloxy)-3-phenylpropionyl-His-[(D)-Manno-Pentol]

Die Titelverbindung wird analog Beispiel 6 synthetisiert.
$R_f$ (Aceton/$H_2O$/konz. $NH_3$ 100:10:5) = 0,15
MS (FAB): 703 (M+1)

**Beispiel 8**

(3-t-Butylsulfonyl,2-thienylmethyl)propionyl-His-[(D)-Manno-Pentol]

Die Titelverbindung wird analog Beispiel 2 synthetisiert.
$R_f$ (Aceton/Wasser 10:1) = 0,50 MS (FAB): 687 (M+1)

Referenzbeispiel 9

(2(S)-Benzyl,3-t-butylsulfonyl)propionyl-His-4(S)-(5-cyclohexyl-1,2,3-trihydroxy)-pentylamid

240 mg (2(S)-Benzyl,3-t-butylsulfonyl)propionyl-His-(DNP)-OH wird mit 69 mg HOBt, 93 mg DCC und 0,3 ml NEM in 8 ml DMF (absolut) gelöst und auf 0°C gekühlt. Danach erfolgt die Zugabe der unter 9a) beschriebenen Lösung von 4(S)-(5-Cyclohexyl-1,2,3-trihydroxy)pentylamin. Nach Aufwärmen verbleibt die Lösung 48 Stunden bei RT.
Die Reaktionslösung wird mit 0,5 ml $H_2O$ versetzt, danach vom entstandenen Dicyclohexylharnstoff abfiltriert und das Filtrat in 25 ml EE aufgenommen. Diese Lösung wird mit 10 % $NaHCO_3$-Lösung, Wasser und gesättigter NaCl-Lösung gewaschen, über Watte filtriert und im Vakuum eingeengt. Dies so erhaltene ölige Rohprodukt wird über Kieselgel mit $CH_2Cl_2$/$CH_3OH$ 20:1 chromatographiert. Das Produkt mit einem $R_f$ von 0.5 ($CH_2Cl_2$/$CH_3OH$ 9:1 auf Kieselgel) wird in einer Ausbeute von 60 mg als glasartiger Feststoff erhalten.
MS (FAB) 787 (M+1)

Das Produkt wird in 5 ml $CH_2Cl_2$ (absolut) gelöst und mit 0,2 ml Thiophenol 4 Stunden bei RT gerührt. Nach Abfiltrieren des Lösungsmittels im Vakuum wird das Rohgemisch mit $CH_2Cl_2$/$CH_3OH$ 20:1 an Kieselgel chromatographiert. Man erhält 16 mg der Titelverbindung.
MS (FAB): 621 (M+1)
$R_f$ ($CH_2Cl_2$/MeOH 9:1) = 0,25

a) 4(S)-(5-Cyclohexyl-1,2,3-trihydroxy)-pentylamin

130 mg (2RS,3RS,4S)-4-tert.-Butoxycarbonylamino-5-cyclohexyl-1,2,3-trihydroxypentan (Beispiel 9b) werden in 2 ml DME gelöst und mit 4 ml ges. HCl/DME-Lösung versetzt und 30 Min. bei 0°C gerührt. Nach Aufwärmen auf RT wird die Lösung im Vakuum eingeengt und dreimal jeweils nach Zugabe von Toluol (absolut) eingeengt. Dieses so gewonnene Öl wird sofort in dem folgenden Reaktionsschritt als Lösung in DMF eingesetzt.

b) (2RS,3RS,4S)-4-tert.-Butoxycarbonylamino-5-cyclohexyl-1,2,3-trihydroxypentan

293 mg des in Beispiel 9c) erhaltenen Pentenols werden in 10 ml THF (absolut) zusammen mit 240 mg Trimethylamin-N-oxid aufgelöst und mit 11 mg Osmiumtetroxid versetzt. Die Lösung verfärbt sich nach wenigen Minuten grünbraun und wird über Nacht bei RT gerührt. Die Lösung wird mit 10 % $NaHSO_3$-Lösung versetzt; 30 Minuten gerührt, danach im Vakuum eingeengt, mit 50 ml Essigester aufgenommen und von der wäßrigen Phase getrennt. Die organische Phase wird mit 1N-HCl, 10 %iger $Na_2SO_4$ gewaschen, getrocknet und im Vakuum eingeengt. Es verbleibt ein amorpher Feststoff von 140 mg.
MS (DCI): 318 (M+1)

c) 4S-tert-Butoxycarbonylamino-5-cyclohexyl-cis-2-pentenol

In 20 ml absolutem Methylenchlorid wurden 0,98 g 4S-tert-Butoxycarbonylamin-5-cyclohexyl-cis-2-pentensäure (hergestellt nach W.C. Still et al. Tetrahedron Lett. 1983, 4405) bei -78°C mit 5,5 ml einer 1,2 M-Lösung von Diisobutylaluminiumhydrid in Toluol versetzt. Nach 1 Stunde läßt man auf RT aufwärmen. Zugabe von 1 ml Methanol beendet die Reaktion und nach Verdünnen mit 80 ml $CH_2Cl_2$ wird die Lösung jeweils einmal gegen 10 % K-Na-Tartratlösung, 10 % Zitronensäurelösung und gesättigter NaCl-Lösung geschüttelt. Die Lösung wird über $MgSO_4$ getrocknet, im Vakuum eingeengt und das erhaltene ölige Rohprodukt über Kieselgel (Cyclohexan/Essigester) chromatographiert. Es werden 480 mg eines leicht gelben Öles erhalten.

**Beispiel 10**

Iva-Phe-Nva-[(D)-Manno-pentol]

Die Verbindung wird analog Beispiel 1) und 1a) aus Iva-Phe-Nva-OH und H-(D)-Manno-Pentol(XX) hergestellt.

$R_f$ ($CH_2Cl_2$/MeOH) = 0,06 MS (FAB + LiI): 614 (M+Li)

Die Titelverbindungen der Beispielen 11 und 12 werden analog Beispiel 2 hergestellt:

**Beispiel 11**

Indolyl-2-carbonyl-His-[(D)-Nanno-Pentol]

$R_f$ ($CH_2CH_2$:MeOH:konz. wäßr.$NH_3$ = 50:10:1) = 0,10
MS (FAB): 558 (M+1)

**Beispiel 12**

2(S)-Hydroxy,3-phenylpropionyl-His-[(D)-Manno-Pentol]

$R_f$ (Aceton/$H_2O$ 10:1) = 0,25 MS (FAB): 563 (M+1)

## Patentansprüche

**Patentansprüche für folgende Vertragsstaaten : AT, BE, CH, DK, DE, FR, GB, IT, LI, LU, NL, SE**

1. Verbindung der Formel I

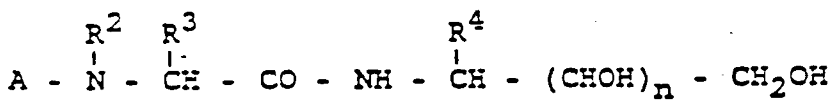

(I)

in welcher
A einen Rest der Formeln II, III oder IV bedeutet

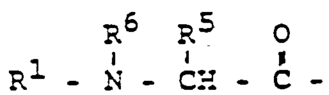

(II)

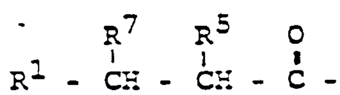

(III)

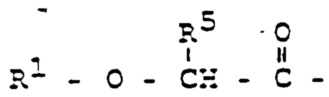 (IV)

| | |
|---|---|
| $R^1$ | Wasserstoff, $(C_1-C_8)$-Alkylsulfonyl; $(C_1-C_8)$-Alkylsulfinyl; 2-Hydroxy-ethylsulfonyl; 2-Hydroxy-propylsulfonyl; 2-Hydroxypropionyl; 3-Hydroxypropionyl; 3-Hydroxybutyryl; 2-Hydroxy-3-methylbutyryl; $(C_1-C_8)$-Alkanoyloxy-$(C_1-C_{10})$-alkyl; n-Decanoyl; Formyl; Acetyl; Propionyl; Pivaloyl; Isovaleryl; Isobutyryl; (3-Amino-3,3-dimethyl)-propionyl; 4-Aminobutyryl; 5-Aminopentanoyl; 6-Aminohexanoyl; Dimethylaminoacetyl; Piperidino-4-carbonyl; Morpholino-4-carbonyl; Cyclopropylcarbonyl; Cyclobutylcarbonyl; Cyclopentylcarbonyl; Cyclohexylcarbonyl; 3-Aminocyclobutylcarbonyl; 4-Aminocyclohexylcarbonyl; 3-Aminocyclobutylsulfonyl; 4-Aminocyclohexylsulfonyl; Phenylacetyl; Phenylpropanoyl; Phenylbutanoyl; 2-Pyridyl-$(C_1-C_8)$-alkanoyl; 3-Pyridyl-$(C_1-C_8)$-alkanoyl; 4-Pyridyl-$(C_1-C_8)$-alkanoyl; 4-Chlorbenzoyl; 4-Methylbenzoyl; 2-Methoxycarbonylbenzoyl; 4-Methoxybenzoyl; Pyrrolyl-2-carbonyl; Pyridyl-3-carbonyl; Benzolsulfonyl; Methoxycarbonyl; Ethoxycarbonyl; Isobutoxycarbonyl; tert.-Butoxycarbonyl; 2-(Tri-methylsilyl)-ethoxycarbonyl; 2,2,2-Trichlor-ethoxycarbonyl; 1,1-Dimethyl-2,2,2-trichlorethoxy-carbonyl; Benzyloxy-carbonyl; 1- oder 2-Naphthylmethoxycarbonyl; 9-Fluorenylmethoxycarbonyl; 4-Amino-piperidino-1-carbonyl; 4-Aminomethylpiperidino-1-carbonyl; N-Methyl-[2-<N-(morpholinocarbonyl)-N-methylamino>-ethyl]-aminocarbonyl; oder N-(4-piperidino)-carbamoyl bedeutet; oder |
| $R^1$ und $R^5$ | zusammen mit dem sie tragenden Stickstoffatom einen 8- bis 12-gliedrigen bicyclischen Ring bilden, der aromatisch, teilhydriert oder vollständig hydriert sein kann; |
| $R^2$ und $R^6$ | unabhängig voneinander besonders Wasserstoff oder Methyl bedeuten; |
| $R^3$ und $R^5$ | unabhängig voneinander Wasserstoff, Methyl, Ethyl, Isopropyl, n-Propyl, n-Butyl, Isobutyl, sec.-Butyl, 3-Guanidinopropyl, Carbamoylmethyl, 2-Carbamoylethyl, Carboxymethyl, 2-Carboxyethyl, Mercaptomethyl, 2-(Methylthio)-ethyl, (1-Mercapto-1-methyl)-ethyl, Hydroxymethyl, 1-Hydroxyethyl, Aminomethyl, 2-Aminoethyl, 3-Aminopropyl, 4-Aminobutyl, N,N-Dimethylamino, Cyclohexylmethyl, Imidazol-4-yl-methyl, Benzyl, 2-Methyl-benzyl, 3-Methylbenzyl, Indol-3-yl-methyl, 4-Hydroxybenzyl, 4-Methoxybenzyl, 3,4-Dihydroxybenzyl, 3,4-Dimethoxybenzyl, (Benzdioxolan-5-yl)-methyl, 2-Thienyl, 2-Thienylmethyl, 2-(2-Thienyl)-ethyl, 3-Thienyl, 3-Thienylmethyl, 2-(3-Thienyl)-ethyl, 4-Chlorbenzyl, 2-(Methylsulfinyl)-ethyl; 2-(Methylsulfonyl)-ethyl, 2-Pyridylmethyl, 3-Pyridylmethyl, 4-Pyridylmethyl, Cyclohexyl, (1-Methyl-imidazol-4-yl)-methyl, (3-Methyl-imidazol-4-yl)-methyl, Phenyl, 1-Naphthylmethyl, 2-Naphthylmethyl, 2-Phenylethyl, 2-Thiazolylmethyl, 4-Thiazolylmethyl, 3-Pyrazolylmethyl, 4-Pyrimidinylmethyl, Indol-2-yl-methyl, 2-Benzo[b]thienylmethyl, 3-Benzo[b]thienylmethyl, 2-Furylmethyl, Cyclohexyl oder Cyclopentyl bedeuten; |
| $R^4$ | $(C_3-C_{12})$-Alkyl; mono- oder bicyclisches $(C_3-C_{12})$-Cycloalkyl oder $(C_3-C_{12})$ Cycloalkylmethyl, wobei der Cycloalkylteil gegebenenfalls durch $(C_1-C_4)$-Alkyl substituiert ist; $(C_6-C_{10})$-Aryl-methyl; Dithiolanyl; Dithiolanylmethyl; Dithianyl oder Dithianylmethyl bedeutet; |
| $R^7$ | Wasserstoff bedeutet oder zusammen mit $R^1$ und den diese tragenden Atomen ein mono- oder bicyclisches gesättigtes oder teilweise ungesättigtes Ringsystem mit 5 - 12 Ringgliedern, das außer Kohlenstoff noch 1 Schwefelatom enthält, welches besonders bevorzugt zum Sulfon oxidiert ist bildet, oder zusammen mit $R^5$ und den diese tragenden Atome ein Thiochromansystem, dessen Schwefelatom besonders bevorzugt zum Sulfon oxidiert ist, bildet und |
| n | 3 - 6 bedeutet sowie deren physiologisch verträgliche Salze. |

**2.** Verfahren zur Herstellung einer Verbindung der Formel (I) gemäß Anspruch 1 , dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

**3.** Verbindung der Formel (I) gemäß Anspruch 1 zur Anwendung als Heilmittel.

4. Verbindung der Formel (I) gemäß Anspruch 1 zur Anwendung als Heilmittel bei der Behandlung des Bluthochdrucks und der kongestiven Herzinsuffizienz.

5. Pharmazeutische Zubereitung enthaltend eine Verbindung der Formel (I) gemäß Anspruch 1.

6. Verfahren zur Herstellung einer Zubereitung gemäß Anspruch 5, dadurch gekennzeichnet, daß man den Wirkstoff der Formel (I) zusammen mit einem physiologisch annehmbaren Träger und gegebenenfalls weiteren Zusatz-, Hilfs- und/oder Konservierungsstoffen in eine geeignete Darreichungsform bringt.

**Patentansprüche für folgende Vertragsstaaten : ES, GR**

1. Verfahren zur Herstellung einer Verbindung der Formel I

$$A - \overset{R^2}{\overset{|}{N}} - \overset{R^3}{\overset{|}{CH}} - CO - NH - \overset{R^4}{\overset{|}{CH}} - (CHOH)_n - CH_2OH \qquad (I)$$

in welcher

A einen Rest der Formeln II, III oder IV bedeutet

$$R^1 - \overset{R^6}{\overset{|}{N}} - \overset{R^5}{\overset{|}{CH}} - \overset{O}{\overset{\|}{C}} - \qquad (II)$$

$$R^1 - \overset{R^7}{\overset{|}{CH}} - \overset{R^5}{\overset{|}{CH}} - \overset{O}{\overset{\|}{C}} - \qquad (III)$$

$$R^1 - O - \overset{R^5}{\overset{|}{CH}} - \overset{O}{\overset{\|}{C}} - \qquad (IV)$$

$R^1$ Wasserstoff, ($C_1$-$C_8$)-Alkylsulfonyl; ($C_1$-$C_8$)-Alkyl- sulfinyl; 2-Hydroxy-ethylsulfonyl; 2-Hydroxy-propylsulfonyl; 2-Hydroxypropionyl; 3-Hydroxypropionyl; 3-Hydroxybutyryl; 2-Hydroxy-3-methylbutyryl; ($C_1$-$C_8$)-Alkanoyloxy-($C_1$-$C_{10}$)-alkyl; n-Decanoyl; Formyl; Acetyl; Propionyl; Pivaloyl; Isovaleryl; Isobutyryl; (3-Amino-3,3-dimethyl)-propionyl; 4-Aminobutyryl; 5-Aminopentanoyl; 6-Aminohexanoyl; Dimethylaminoacetyl; Piperidino-4-carbonyl; Morpholino-4-carbonyl; Cyclopropylcarbonyl; Cyclobutylcarbonyl; Cyclopentylcarbonyl; Cyclohexylcarbonyl; 3-Aminocyclobutylcarbonyl; 4-Aminocyclohexylcarbonyl; 3-Aminocyclobutylsulfonyl; 4-Aminocyclohexylsulfonyl; Phenylacetyl; Phenylpropanoyl; Phenylbutanoyl; 2-Pyridyl-($C_1$-$C_8$)-alkanoyl; 3-Pyridyl-($C_1$-$C_8$)-alkanoyl; 4-Pyridyl-($C_1$-$C_8$)-alkanoyl; 4-Chlorbenzoyl; 4-Methylbenzoyl; 2-Methoxycarbonylbenzoyl; 4-Methoxybenzoyl; Pyrrolyl-2-carbonyl; Pyridyl-3-carbonyl; Benzolsulfonyl; Methoxycarbonyl; Ethoxycarbonyl; Isobutoxycarbonyl; tert.-Butoxycarbonyl; 2-(Tri-methylsilyl)-ethoxycarbonyl; 2,2,2-Trichlor-ethoxycarbonyl; 1,1-Dimethyl-2,2,2-trichlorethoxy-carbonyl; Benzyloxy-carbonyl; 1- oder 2-Naphthylmethoxycarbonyl; 9-Fluorenylmethoxycarbonyl; 4-Amino-piperidino-1-carbonyl; 4-Aminomethylpiperidino-1-carbonyl; N-Methyl-[2-<N-(morpholinocarbonyl)-N-methylamino>-ethyl]-aminocarbonyl; oder N-(4-piperidino)-carbamoyl bedeutet;

$R^1$ und $R^5$ zusammen mit dem sie tragenden Stickstoffatom einen 8- bis 12-gliedrigen bicyclischen Ring bilden, der aromatisch, teilhydriert oder vollständig hydriert sein kann;

| | |
|---|---|
| $R^2$ und $R^6$ | unabhängig voneinander besonders Wasserstoff oder Methyl bedeuten; |
| $R^3$ und $R^5$ | unabhängig voneinander Wasserstoff, Methyl, Ethyl, Isopropyl, n-Propyl, n-Butyl, Isobutyl, sec.-Butyl, 3-Guanidinopropyl, Carbamoylmethyl, 2-Carbamoylethyl, Carboxymethyl, 2-Carboxyethyl, Mercaptomethyl, 2-(Methylthio)-ethyl, (1-Mercapto-1-methyl)-ethyl, Hydroxymethyl, 1-Hydroxyethyl, Aminomethyl, 2-Aminoethyl, 3-Aminopropyl, 4-Aminobutyl, N,N-Dimethylamino, Cyclohexylmethyl, Imidazol-4-yl-methyl, Benzyl, 2-Methyl-benzyl, 3-Methylbenzyl, Indol-3-yl-methyl, 4-Hydroxybenzyl, 4-Methoxybenzyl, 3,4-Dihydroxybenzyl, 3,4-Dimethoxybenzyl, (Benzdioxolan-5-yl)-methyl, 2-Thienyl, 2-Thienylmethyl, 2-(2-Thienyl)-ethyl, 3-Thienyl, 3-Thienylmethyl, 2-(3-Thienyl)-ethyl, 4-Chlorbenzyl, 2-(Methylsulfinyl)-ethyl, 2-(Methylsulfonyl)-ethyl, 2-Pyridylmethyl, 3-Pyridylmethyl, 4-Pyridylmethyl, Cyclohexyl, (1-Methyl-imidazol-4-yl)-methyl, (3-Methyl-imidazol-4-yl)-methyl, Phenyl, 1-Naphthylmethyl, 2-Naphthylmethyl, 2-Phenylethyl, 2-Thiazolylmethyl, 4-Thiazolylmethyl, 3-Pyrazolylmethyl, 4-Pyrimidinylmethyl, Indol-2-yl-methyl, 2-Benzo[b]thienylmethyl, 3-Benzo[b]-thienylmethyl, 2-Furylmethyl, Cyclohexyl oder Cyclopentyl bedeuten; |
| $R^4$ | $(C_3-C_{12})$-Alkyl; mono- oder bicyclisches $(C_3-C_{12})$-Cycloalkyl oder $(C_3-C_{12})$-Cycloalkylmethyl, wobei der Cycloalkylteil gegebenenfalls durch $(C_1-C_4)$-Alkyl substituiert ist; $(C_6-C_{10})$-Aryl-methyl; Dithiolanyl; Dithiolanylmethyl; Dithianyl oder Dithianylmethyl bedeutet; |
| $R^7$ | Wasserstoff bedeutet oder zusammen mit $R^1$ und den diese tragenden Atomen ein mono- oder bicyclisches gesättigtes oder teilweise ungesättigtes Ringsystem mit 5 - 12 Ringgliedern, das außer Kohlenstoff noch 1 Schwefelatom enthält, welches besonders bevorzugt zum Sulfon oxidiert ist bildet, oder zusammen mit $R^5$ und den diese tragenden Atome ein Thiochromansystem, dessen Schwefelatom besonders bevorzugt zum Sulfon oxidiert ist, bildet und |
| n | 3 - 6 bedeutet |

sowie deren physiologisch verträgliche Salze, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

2. Verfahren zur Herstellung einer pharmazeutischen Zubereitung enthaltend eine Verbindung gemäß Anspruch 1 dadurch gekennzeichnet, daß man den Wirkstoff zusammen mit einem physiologisch annehmbaren Träger und gegebenenfalls weiteren Zusatz-, Hilfs- und/oder Konservierungsstoffen in eine geeignete Darreichungsform bringt.

## Claims

### Claims for the following Contracting States : AT, BE, CH, DE, FR, GB, IT, LI, LU, NL, SE, DK

1. A compound of the formula I

$$A - \overset{R^2}{\overset{|}{N}} - \overset{R^3}{\overset{|}{CH}} - CO - NH - \overset{R^4}{\overset{|}{CH}} - (CHOH)_n - CH_2OH \qquad (I)$$

in which

| | |
|---|---|
| A | denotes a radical of the formula II, III or IV |

$$R^1 - \overset{R^6}{\overset{|}{N}} - \overset{R^5}{\overset{|}{CH}} - \overset{O}{\overset{\|}{C}} - \qquad (II)$$

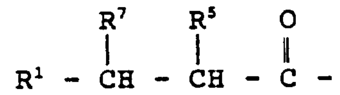 (III)

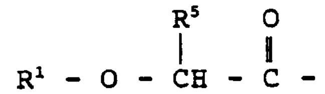 (IV),

$R^1$ denotes hydrogen; $(C_1\text{-}C_8)$-alkylsulfonyl; $(C_1\text{-}C_8)$-alkylsulfinyl; 2-hydroxy-ethylsulfonyl; 2-hydroxypropylsulfonyl; 2-hydroxypropionyl; 3-hydroxypropionyl; 3-hydroxybutyryl; 2-hydroxy-3-methylbutyryl; $(C_1\text{-}C_8)$-alkanoyloxy-$(C_1\text{-}C_{10})$-alkyl; n-decanoyl; formyl; acetyl; propionyl; pivaloyl; isovaleryl; isobutyryl; (3-amino-3,3-dimethyl)-propionyl; 4-aminobutyryl; 5-aminopentanoyl; 6-aminohexanoyl; dimethylaminoacetyl; piperidino-4-carbonyl; morpholino-4-carbonyl; cyclopropylcarbonyl; cyclobutylcarbonyl; cyclopentylcarbonyl; cyclohexylcarbonyl; 3-aminocyclobutylcarbonyl; 4-aminocyclohexylcarbonyl; 3-aminocyclobutylsulfonyl; 4-aminocyclohexylsulfonyl; phenylacetyl; phenylpropanoyl; phenylbutanoyl; 2-pyridyl-$(C_1\text{-}C_8)$-alkanoyl; 3-pyridyl-$(C_1\text{-}C_8)$-alkanoyl; 4-pyridyl-$(C_1\text{-}C_8)$-alkanoyl; 4-chlorobenzoyl; 4-methylbenzoyl; 2-methoxycarbonylbenzoyl; 4-methoxybenzoyl; 2-pyrrolylcarbonyl; 3-pyridylcarbonyl; benzenesulfonyl; methoxycarbonyl; ethoxycarbonyl; isobutoxycarbonyl; tert.-butoxycarbonyl; 2-(trimethylsilyl)-ethoxycarbonyl; 2,2,2-trichloroethoxycarbonyl ; 1,1-dimethyl-2,2,2-trichloroethoxycarbonyl; benzyloxycarbonyl; 1- or 2-naphthylmethoxycarbonyl; 9-fluorenylmethoxycarbonyl; 4-amino-piperidino-1-carbonyl; 4-aminomethyl-piperidino-1-carbonyl; N-methyl{2-[N-(morpholino-carbonyl)-N-methylamino]ethyl}-aminocarbonyl; or N-(4-piperidino)-carbamoyl;

$R^1$ and $R^5$ together with the nitrogen atom carrying them form an 8- to 12-membered bicyclic ring system which may be aromatic, partially hydrogenated or completely hydrogenated;

$R^2$ and $R^6$ independently of one another denote particularly hydrogen or methyl;

$R^3$ and $R^5$ independently of one another denote hydrogen, methyl, ethyl, isopropyl, n-propyl, n-butyl, isobutyl, sec.-butyl, 3-guanidinopropyl, carbamoylmethyl, 2-carbamoylethyl, carboxymethyl, 2-carboxyethyl, mercaptomethyl, 2-(methylthio)-ethyl, (1-mercapto-1-methyl)-ethyl, hydroxymethyl, 1-hydroxyethyl, aminomethyl, 2-aminoethyl, 3-aminopropyl, 4-aminobutyl, N,N-dimethylamino, cyclohexylmethyl, 4-imidazolyl-methyl, benzyl, 2-methylbenzyl, 3-methylbenzyl, 3-indolyl-methyl, 4-hydroxybenzyl, 4-methoxybenzyl, 3,4-dihydroxybenzyl, 3,4-dimethoxybenzyl, (benzodioxolan-5-yl)-methyl, 2-thienyl, 2-thienylmethyl, 2-(2-thienyl)-ethyl, 3-thienyl, 3-thienylmethyl, 2-(3-thienyl)-ethyl, 4-chlorobenzyl, 2-(methylsulfinyl)-ethyl, 2-(methylsulfonyl)-ethyl, 2-pyridylmethyl, 3-pyridylmethyl, 4-pyridylmethyl, (1-methyl-4-imidazolyl)-methyl, (3-methyl-4-imidazolyl)-methyl, phenyl, 1-naphthylmethyl, 2-naphthylmethyl, 2-phenylethyl, 2-thiazolylmethyl, 4-thiazolylmethyl, 3-pyrazolylmethyl, 4-pyrimidinylmethyl, 2-indolyl-methyl, 2-benzo[b]-thienyl-methyl, 3-benzo[b]thienylmethyl, 2-furylmethyl, cyclohexyl or cyclopentyl;

$R^4$ denotes $(C_3\text{-}C_{12})$-alkyl; mono- or bicyclic $(C_3\text{-}C_{12})$-cycloalkyl or $(C_3\text{-}C_{12})$-cycloalkylmethyl, in which the cycloalkyl part is optionally substituted by $(C_1\text{-}C_4)$-alkyl ; $(C_6\text{-}C_{10})$-aryl-methyl; dithiolanyl; dithiolanylmethyl; dithianyl or dithianylmethyl;

$R^7$ denotes hydrogen or, together with $R^1$ and the atoms carrying these, forms a mono- or bicyclic saturated or partly unsaturated ring system having 5 - 12 ring members, which, in addition to carbon, also contains 1 sulfur atom, which is particularly preferably oxidized to sulfone, or together with $R^5$ and the atoms carrying these forms a thiochroman system, the sulfur atom of which is particularly preferably oxidized to sulfone, and

n denotes 3 - 6,

or a physiologically tolerated salt thereof.

2. A process for the preparation of a compound of the formula (I) as claimed in claim 1, which comprises coupling a fragment having a terminal carboxyl group or a reactive derivative thereof with a corresponding fragment having a free amino group, if appropriate splitting off (a) protective group(s) temporarily introduced to protect further functional groups, and if appropriate converting the compound thus obtained into its physiologically tolerated salt.

3. A compound of the formula (I) as claimed in claim 1 for use as a medicine.

4. A compound of the formula (I) as claimed in claim 1 for use as a medicine in the treatment of high blood pressure and congestive heart failure.

5. A pharmaceutical preparation containing a compound of the formula (I) as claimed in claim 1.

6. A process for producing a preparation as claimed in claim 5, which comprises bringing the active substance of the formula (I), together with a physiologically acceptable excipient and, if appropriate, other additives, auxiliaries and/or preservatives, into a suitable administration form.

**Claims for the following Contracting States : ES, GR**

1. A process for the preparation of a compound of the formula I

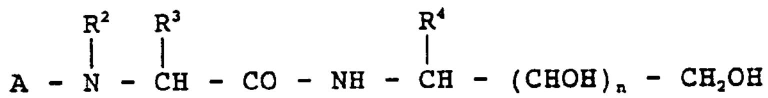

(I)

in which

A denotes a radical of the formula II, III or IV

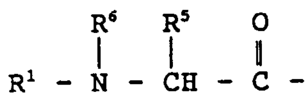

(II)

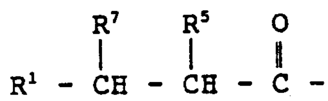

(III)

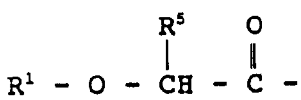

(IV),

$R^1$ denotes hydrogen; ($C_1$-$C_8$)-alkylsulfonyl; ($C_1$-$C_8$)-alkylsulfinyl; 2-hydroxy-ethylsulfonyl; 2-hydroxypropylsulfonyl; 2-hydroxypropionyl; 3-hydroxypropionyl; 3-hydroxybutyryl; 2-hydroxy-3-methylbutyryl; ($C_1$-$C_8$)-alkanoyloxy-($C_1$-$C_{10}$)-alkyl; n-decanoyl; formyl; acetyl; propionyl; pivaloyl; isovaleryl; isobutyryl; (3-amino-3,3-dimethyl)-propionyl; 4-aminobutyryl; 5-aminopentanoyl; 6-aminohexanoyl; dimethylaminoacetyl; piperidino-4-carbonyl; morpholino-4-carbonyl; cyclopropylcarbonyl; cyclobutylcarbonyl; cyclopentylcarbonyl; cyclohexylcarbonyl; 3-aminocyclobutylcarbonyl; 4-aminocyclohexylcarbonyl; 3-aminocyclobutylsulfonyl; 4-aminocyclohexylsulfonyl; phenylacetyl; phenylpropanoyl; phenylbutanoyl; 2-pyridyl-($C_1$-$C_8$)-alkanoyl; 3-pyridyl-($C_1$-$C_8$)-alkanoyl; 4-pyridyl-($C_1$-$C_8$)-alkanoyl; 4-chlorobenzoyl; 4-methylbenzoyl; 2-methoxycarbonylbenzoyl; 4-methoxybenzoyl; 2-pyrrolylcarbonyl; 3-pyridylcarbonyl; benzenesulfonyl; methoxycarbonyl; ethoxycarbonyl; isobutoxycarbonyl; tert.-butoxycarbonyl; 2-(trimethylsilyl)-ethoxycarbonyl; 2,2,2-trichloroethoxycarbonyl; 1,1-dimethyl-2,2,2-trichloroethoxycarbonyl; benzyloxycarbonyl; 1- or 2-naphthylmethoxycarbonyl; 9-fluorenylmethoxycarbonyl; 4-amino-piperidino-1-carbonyl; 4-aminomethyl-piperidino-1-carbonyl; N-methyl{2-[N-(morpholino-carbonyl)-N-methylamino]ethyl}-aminocarbonyl; or N-(4-piperidino)-carbamoyl;

$R^1$ and $R^5$ together with the nitrogen atom carrying them form an 8- to 12-membered bicyclic ring system which may be aromatic, partially hydrogenated or completely hydrogenated;

$R^2$ and $R^6$ independently of one another denote particularly hydrogen or methyl;

$R^3$ and $R^5$ independently of one another denote hydrogen, methyl, ethyl, isopropyl, n-propyl, n-butyl, isobutyl, sec.-butyl, 3-guanidinopropyl, carbamoylmethyl, 2-carbamoylethyl, carboxymethyl, 2-carboxyethyl, mercaptomethyl, 2-(methylthio)-ethyl, (1-mercapto-1-methyl)-ethyl, hydroxymethyl, 1-hydroxyethyl, aminomethyl, 2-aminoethyl, 3-aminopropyl, 4-aminobutyl, N,N-dimethylamino, cyclohexylmethyl, 4-imidazolyl-methyl, benzyl, 2-methylbenzyl, 3-methylbenzyl, 3-indolyl-methyl, 4-hydroxybenzyl, 4-methoxybenzyl, 3,4-dihydroxybenzyl, 3,4-dimethoxybenzyl, (benzodioxolan-5-yl)-methyl, 2-thienyl, 2-thienylmethyl, 2-(2-thienyl)-ethyl, 3-thienyl, 3-thienylmethyl, 2-(3-thienyl)-ethyl, 4-chlorobenzyl, 2-(methylsulfinyl)-ethyl, 2-(methylsulfonyl)-ethyl, 2-pyridylmethyl, 3-pyridylmethyl, 4-pyridylmethyl, (1-methyl-4-imidazolyl)-methyl, (3-methyl-4-imidazolyl)-methyl, phenyl, 1-naphthylmethyl, 2-naphthylmethyl, 2-phenylethyl, 2-thiazolylmethyl, 4-thiazolylmethyl, 3-pyrazolylmethyl, 4-pyrimidinylmethyl, 2-indolyl-methyl, 2-benzo[b]-thienyl-methyl, 3-benzo[b]thienylmethyl, 2-furylmethyl, cyclohexyl or cyclopentyl;

$R^4$ denotes ($C_3$-$C_{12}$)-alkyl; mono- or bicyclic ($C_3$-$C_{12}$)-cycloalkyl or ($C_3$-$C_{12}$)-cycloalkylmethyl, in which the cycloalkyl part is optionally substituted by ($C_1$-$C_4$)-alkyl; ($C_6$-$C_{10}$)-aryl-methyl; dithiolanyl; dithiolanylmethyl; dithianyl or dithianylmethyl;

$R^7$ denotes hydrogen or, together with $R^1$ and the atoms carrying these, forms a mono- or bicyclic saturated or partly unsaturated ring system having 5 - 12 ring members, which, in addition to carbon, also contains 1 sulfur atom, which is particularly preferably oxidized to sulfone, or together with $R^5$ and the atoms carrying these forms a thiochroman system, the sulfur atom of which is particularly preferably oxidized to sulfone, and

n denotes 3 - 6,

or a physiologically tolerated salt thereof,

which comprises coupling a fragment having a terminal carboxyl group or a reactive derivative thereof with a corresponding fragment having a free amino group, if appropriate splitting off (a) protective group(s) temporarily introduced to protect further functional groups, and if appropriate converting the compound thus obtained into its physiological tolerated salt.

2. A process for producing a pharmaceutical preparation containing a compound as claimed in claim 1, which comprises bringing the active substance of the formula (I), together with a physiologically acceptable excipient and, if appropriate, other additives, auxiliaries and/or preservatives, into a suitable administration form.

## Revendications

## Revendications pour les Etats contractants suivants : AT, BE, CH, DE, DK, FR, GB, IT, LI, NL, SE

1. Composé de formule I

$$A - \overset{R^2}{\underset{|}{N}} - \overset{R^3}{\underset{|}{CH}} - CO - NH - \overset{R^4}{\underset{|}{CH}} - (CHOH)_n - CH_2OH \qquad (I)$$

dans laquelle

A représente un radical de formules II, III ou IV

$$R^1 - \overset{R^6}{N} - \overset{R^5}{CH} - \overset{O}{\overset{\|}{C}} - \qquad (II)$$

$$R^1 - \overset{R^7}{CH} - \overset{R^5}{CH} - \overset{O}{\overset{\|}{C}} - \qquad (III)$$

$$R^1 - O - \overset{R^5}{\overset{|}{CH}} - \overset{O}{\overset{\|}{C}} - \qquad (IV)$$

$R^1$ représente un atome d'hydrogène ou un groupe alkyl($C_1$-$C_8$)- sulfonyle, alkyl($C_1$-$C_8$)-sulfinyle, 2-hydroxyéthylsulfonyle, 2-hydroxypropylsulfonyle, 2-hydroxypropionyle, 3-hydroxypropionyle, 3-hydroxybutyryle, 2-hydroxy-3-méthylbutyryle, alcanoyloxy($C_1$-$C_8$)-alkyle($C_1$-$C_{10}$), n-décanoyle, formyle, acétyle, propionyle, pivaloyle, isovaléryle, isobutyryle, (3-amino-3,3-diméthyl)-propionyle, 4-aminobutyryle, 5-aminopentanoyle, 6-aminohexanoyle, diméthylaminoacétyle, pipéridino-4-carbonyle, morpholino-4-carbonyle, cyclopropylcarbonyle, cyclobutylcarbonyle, cyclopentylcarbonyle, cyclohexylcarbonyle, 3-aminocyclobutylcarbonyle, 4-aminocyclohexylcarbonyle, 3-aminocyclobutylsulfonyle, 4-aminocyclohexylsulfonyle, phénylacétyle, phénylpropanoyle, phénylbutanoyle, 2-pyridyl-alcanoyle-($C_1$-$C_8$), 3-pyridyl-alcanoyle($C_1$-$C_8$), 4-pyridyl-alcanoyl-($C_1$-$C_8$), 4-chlorobenzoyle, 4-méthylbenzoyle, 2-méthoxycarbonylbenzoyle, 4-méthoxybenzoyle, pyrrolyl-2-carbonyle, pyridyl-3-carbonyle, benzènesulfonyle, méthoxycarbonyle, éthoxycarbonyle, isobutoxycarbonyle, tert-butoxycarbonyle, 2-(triméthylsilyl)-éthoxycarbonyle, 2,2,2-trichloréthoxycarbonyle, 1,1-diméthyl-2,2,2-trichloréthoxycarbonyle, benzyloxycarbonyle, 1- ou 2-naphtylméthoxycarbonyle, 9-fluorénylméthoxycarbonyle, 4-aminopipéridino-1-carbonyle, 4-aminométhyl-pipéridino-1-carbonyle, N-méthyl-[2-<N-(morpholinocarbonyl)-N-méthylamino>-éthyl]aminocarbonyle ou N-(4-pipéridino)-carbamoyle;

$R^1$ et $R^5$ forment, conjointement avec l'atome d'azote qui les porte, un cycle bicyclique à 8-12 chaînons qui peut être aromatique, partiellement hydrogéné ou totalement hydrogéné;

$R^2$ et $R^6$ représentent, indépendamment l'un de l'autre, un atome d'hydrogène ou le groupe méthyle;

$R^3$ et $R^5$ représentent, indépendamment l'un de l'autre, un atome d'hydrogène ou le groupe méthyle, éthyle, isopropyle, n-propyle, n-butyle, isobutyle, sec-butyle, 3-guanidinopropyle, carbamoylméthyle, 2-carbamoyléthyle, carboxyméthyle, 2-carboxyéthyle, mercaptométhyle, 2-(méthylthio)-éthyle, (1-mercapto-1-méthyl)-éthyle, hydroxyméthyle, 1-hydroxyéthyle, aminométhyle, 2-aminoéthyle, 3-aminopropyle, 4-aminobutyle, N,N-diméthylamino, cyclohexylméthyle, imidazol-4-yl-méthyle, benzyle, 2-méthylbenzyle, 3-méthylbenzyle, indol-3-yl-méthyle, 4-hydroxybenzyle, 4-méthoxybenzyle, 3,4-dihydroxybenzyle, 3,4-diméthoxybenzyle, (benzodioxolanne-5-yl)-méthyle, 2-thiényle, 2-thiénylméthyle, 2-(2-thiényl)-éthyle, 3-thiényle, 3-thiénylméthyle, 2-(3-thiényl)-éthyle, 4-chlorobenzyle, 2-(méthylsulfinyl)-éthyle, 2-(méthylsulfonyl)-éthyle, 2-pyridylméthyle, 3-pyridylméthyle, 4-pyridylméthyle, (1-méthyl-imidazol-4-yl)-méthyle, (3-méthyl-imidazol-4-yl)-méthyle, phényle, 1-naphtylméthyle, 2-naphtylméthyle, 2-phényléthyle, 2-thiazolylméthyle, 4-thiazolylméthyle, 3-pyrazolylméthyle, 4-pyrimidinylméthyle, indol-2-yl-méthyle, 2-benzo[b]thiénylméthyle, 3-benzo[b]thiénylméthyle, 2-furylméthyle, cyclohexyle ou cyclopentyle;

$R^4$ représente un radical alkyle en $C_3$-$C_{12}$, cycloalkyle en $C_3$-$C_{12}$ mono- ou bicyclique, ou cycloalkyl($C_3$-$C_{12}$)-méthyle, le fragment cycloalkyle étant éventuellement substitué par un groupe alkyle en $C_1$-$C_4$; un radical aryl($C_6$-$C_{10}$)-méthyle; le radical dithiolanyle; le radical dithiolanylméthyle, dithianyle ou dithianylméthyle;

$R^7$ représente un atome d'hydrogène ou forme, conjointement avec $R^1$ et les atomes qui les portent, un système cyclique mono- ou bicyclique, saturé ou partiellement insaturé, ayant 5-12 chaînons formant le(s) cycle(s), qui, outre le carbone, peut contenir encore un atome de soufre qui, de façon particulièrement préférée, est oxydé en la sulfone, ou forme, conjointement avec $R^5$ et les atomes qui les portent, un système thiochromane dont l'atome de soufre est de façon particulièrement préférée oxydé en la sulfone, et

n va de 3 à 6,

ainsi que leurs sels physiologiquement acceptables.

**2.** Procédé pour la préparation d'un composé de formule I selon la revendication 1, caractérisé en ce que l'on assemble un fragment à groupe carboxy terminal, ou un dérivé réactif de celui-ci, avec un fragment correspondant à groupe amino libre, on élimine un(des) groupe(s) protecteur(s) éventuellement introduit(s) temporairement pour la protection d'autres groupes fonctionnels, et éventuellement on convertit le composé obtenu en un de ses sels physiologiquements acceptables.

3. Composé de formule (I) selon la revendication 1, pour utilisation en tant que médicament.

4. Composé de formule (I) selon la revendication 1, pour utilisation en tant que médicament dans le traitement de l'hypertension et de l'insuffisance cardiaque congestive.

5. Composition pharmaceutique contenant un composé de formule (I) selon la revendication 1.

6. Procédé pour la préparation d'une composition selon la revendication 5, caractérisé en ce que l'on met sous une forme d'administration appropriée la substance active de formule (I), conjointement avec un véhicule physiologiquement acceptable et éventuellement d'autres additifs, adjuvants et/ou conservateurs.

**Revendications pour les Etats contractants suivants : ES, GR**

1. Procédé pour la préparation d'un composé de formule I

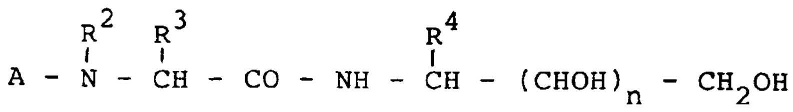

(I)

dans laquelle

A représente un radical de formules II, III ou IV

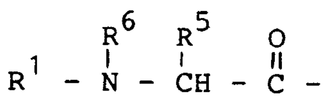

(II)

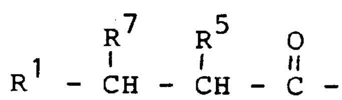

(III)

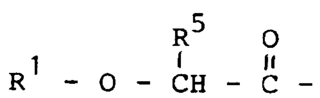

(IV)

$R^1$ représente un atome d'hydrogène ou un groupe alkyl($C_1$-$C_8$) sulfonyle, alkyl($C_1$-$C_8$)-sulfinyle, 2-hydroxyéthylsulfonyle, 2-hydroxypropylsulfonyle, 2-hydroxypropionyle, 3-hydroxypropionyle, 3-hydroxybutyryle, 2-hydroxy-3-méthylbutyryle, alcanoyloxy($C_1$-$C_8$)-alkyle($C_1$-$C_{10}$), n-décanoyle, formyle, acétyle, propionyle, pivaloyle, isovaléryle, isobutyryle, (3-amino-3,3-diméthyl)-propionyle, 4-aminobutyryle, 5-aminopentanoyle, 6-aminohexanoyle, diméthylaminoacétyle, pipéridino-4-carbonyle, morpholino-4-carbonyle, cyclopropylcarbonyle, cyclobutylcarbonyle, cyclopentylcarbonyle, cyclohexylcarbonyle, 3-aminocyclobutylcarbonyle, 4-aminocyclohexylcarbonyle, 3-aminocyclobutylsulfonyle, 4-aminocyclohexylsulfonyle, phénylacétyle, phénylpropanoyle, phénylbutanoyle, 2-pyridyl-alcanoyle-($C_1$-$C_8$), 3-pyridyl-alcanoyle($C_1$-$C_8$), 4-pyridyl-alcanoyl-($C_1$-$C_8$), 4-chlorobenzoyle, 4-méthylbenzoyle, 2-méthoxycarbonylbenzoyle, 4-méthoxybenzoyle, pyrrolyl-2-carbonyle, pyridyl-3-carbonyle, benzènesulfonyle, méthoxycarbonyle, éthoxycarbonyle, isobutoxycarbonyle, tert-butoxycarbonyle, 2-(triméthylsilyl)-éthoxycarbonyle, 2,2,2-trichloréthoxycarbonyle, 1,1-diméthyl-2,2,2-trichloréthoxycarbonyle, benzyloxycarbonyle, 1- ou 2-naphtylméthoxycarbonyle, 9-fluorénylméthoxycarbonyle, 4-aminopipéridino-1-carbonyle, 4-aminométhyl-pipéridino-1-carbonyle, N-méthyl-[2-<N-(morpholinocarbonyl)-N-méthylamino>-éthyl]aminocarbonyle ou N-(4-pipéridino)-carbamoyle;

$R^1$ et $R^5$ forment, conjointement avec l'atome d'azote qui les porte, un cycle bicyclique à 8-12 chaînons qui peut être aromatique, partiellement hydrogéné ou totalement hydrogéné;

$R^2$ et $R^6$ représentent, indépendamment l'un de l'autre, un atome d'hydrogène ou le groupe méthyle;

$R^3$ et $R^5$ représentent, indépendamment l'un de l'autre, un atome d'hydrogène ou le groupe méthyle, éthyle, isopropyle, n-propyle, n-butyle, isobutyle, sec-butyle, 3-guanidinopropyle,

carbamoylméthyle, 2-carbamoyléthyle, carboxyméthyle, 2-carboxyéthyle, mercaptométhyle, 2-(méthylthio)-éthyle, (1-mercapto-1-méthyl)-éthyle, hydroxyméthyle, 1-hydroxyéthyle, aminométhyle, 2-aminoéthyle, 3-aminopropyle, 4-aminobutyle, N,N-diméthylamino, cyclohexylméthyle, imidazol-4-yl-méthyle, benzyle, 2-méthylbenzyle, 3-méthylbenzyle, indol-3-yl-méthyle, 4-hydroxybenzyle, 4-méthoxybenzyle, 3,4-dihydroxybenzyle, 3,4-diméthoxybenzyle, (benzodioxolanne-5-yl)-méthyle, 2-thiényle, 2-thiénylméthyle, 2-(2-thiényl)-éthyle, 3-thiényle, 3-thiénylméthyle, 2-(3-thiényl)-éthyle, 4-chlorobenzyle, 2-(méthylsulfinyl)-éthyle, 2-(méthylsulfonyl)-éthyle, 2-pyridylméthyle, 3-pyridylméthyle, 4-pyridylméthyle, (1-méthyl-imidazol-4-yl)-méthyle, (3-méthyl-imidazol-4-yl)-méthyle, phényle, 1-naphtylméthyle, 2-naphtylméthyle, 2-phényléthyle, 2-thiazolylméthyle, 4-thiazolylméthyle, 3-pyrazolylméthyle, 4-pyrimidinylméthyle, indol-2-yl-méthyle, 2-benzo[b]thiénylméthyle, 3-benzo[b]thiénylméthyle, 2-furylméthyle, cyclohexyle ou cyclopentyle;

$R^4$ représente un radical alkyle en $C_3$-$C_{12}$, cycloalkyle en $C_3$-$C_{12}$ mono- ou bicyclique, ou cycloalkyl($C_3$-$C_{12}$)-méthyle, le fragment cycloalkyle étant éventuellement substitué par un groupe alkyle en $C_1$-$C_4$; un radical aryl($C_6$-$C_{10}$)-méthyle; le radical dithiolanyle; le radical dithiolanylméthyle, dithianyle ou dithianylméthyle;

$R^7$ représente un atome d'hydrogène ou forme, conjointement avec $R^1$ et les atomes qui les portent, un système cyclique mono- ou bicyclique, saturé ou partiellement insaturé, ayant 5-12 chaînons formant le(s) cycle(s), qui, outre le carbone, peut contenir encore un atome de soufre qui, de façon particulièrement préférée, est oxydé en la sulfone, ou forme, conjointement avec $R^5$ et les atomes qui les portent, un système thiochromane dont l'atome de soufre est de façon particulièrement préférée oxydé en la sulfone, et

n va de 3 à 6,

ainsi que de ses sels physiologiquement acceptables, caractérisé en ce que l'on assemble un fragment à groupe carboxy terminal, ou un dérivé réactif de celui-ci, avec un fragment correspondant à groupe amino libre, on élimine un(des) groupe(s) protecteur(s) éventuellement introduit(s) temporairement pour la protection d'autres groupes fonctionnels, et éventuellement on convertit le composé obtenu en un de ses sels physiologiquements acceptables.

**2.** Procédé pour la préparation d'une composition pharmaceutique contenant un composé selon la revendication 1, caractérisé en ce que l'on met sous une forme d'administration appropriée la substance active de formule (I), conjointement avec un véhicule physiologiquement acceptable et éventuellement d'autres additifs, adjuvants et/ou conservateurs.